(19) Europäisches Patentamt
European Patent Office
Office européen des brevets

(11) EP 2 497 084 B1

(12) **EUROPÄISCHE PATENTSCHRIFT**

(45) Veröffentlichungstag und Bekanntmachung des Hinweises auf die Patenterteilung:
**25.12.2013 Patentblatt 2013/52**

(21) Anmeldenummer: **10773308.1**

(22) Anmeldetag: **02.11.2010**

(51) Int Cl.:
***G11B 7/245*** *(2006.01)*

(86) Internationale Anmeldenummer:
**PCT/EP2010/066593**

(87) Internationale Veröffentlichungsnummer:
**WO 2011/054796 (12.05.2011 Gazette 2011/19)**

(54) **AUSWAHLVERFAHREN FÜR ADDITIVE IN PHOTOPOLYMEREN**

Selection method for additives in photopolymers

Procédé de sélection pour additifs dans des photopolymères

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.11.2009 EP 09013764**

(43) Veröffentlichungstag der Anmeldung:
**12.09.2012 Patentblatt 2012/37**

(73) Patentinhaber: **Bayer Intellectual Property GmbH**
**40789 Monheim (DE)**

(72) Erfinder:
- **RÖLLE, Thomas**
**51381 Leverkusen (DE)**
- **BRUDER, Friedrich-Karl**
**47802 Krefeld (DE)**
- **FÄCKE, Thomas**
**51375 Leverkusen (DE)**
- **WEISER, Marc-Stephan**
**51379 Leverkusen (DE)**
- **HÖNEL, Dennis**
**53909 Zülpich-Wichterich (DE)**
- **DIEDRICH, Christian**
**40591 Düsseldorf (DE)**

(74) Vertreter: **BIP Patents**
**c/o Bayer Intellectual Property GmbH**
**Creative Campus Monheim**
**Alfred-Nobel-Straße 10**
**40789 Monheim (DE)**

(56) Entgegenhaltungen:
**EP-A- 1 091 267** **JP-A- 2007 101 681**
**US-A1- 2005 259 303**

- **CHANDOLA ET AL: "A QSPR for the plasticization efficiency of polyvinylchloride plasticizers", JOURNAL OF MOLECULAR GRAPHICS AND MODELLING, ELSEVIER SCIENCE, NEW YORK, NY, US, Bd. 26, Nr. 5, 6. Dezember 2007 (2007-12-06), Seiten 824-828, XP022379392, ISSN: 1093-3263**
- **SHELDON T J ET AL: "Pure component properties from group contribution: Hydrogen-bond basicity, hydrogen-bond acidity, Hildebrand solubility parameter, macroscopic surface tension, dipole moment, refractive index and dielectric constant", FLUID PHASE EQUILIBRIA, ELSEVIER, Bd. 231, Nr. 1, 1. April 2005 (2005-04-01) , Seiten 27-37, XP004877786, ISSN: 0378-3812**

Bemerkungen:
Die Akte enthält technische Angaben, die nach dem Eingang der Anmeldung eingereicht wurden und die nicht in dieser Patentschrift enthalten sind.

EP 2 497 084 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die Erfindung betrifft ein Verfahren zur Auswahl von Verbindungen, die als Additive in Photopolymer-Formulierungen zur Herstellung von hellen holographischen Medien verwendet werden können, sowie Photopolymer-Formulierungen welche wenigstens einen Weichmacher enthalten, der nach dem erfindungsgemäßen Verfahren ausgewählt wurde.

In der WO 2008/125229 A1 sind Photopolymer-Formulierungen der eingangs genannten Art beschrieben. Diese umfassen Polyurethan-basierte Matrixpolymere, Schreibmomomere auf Acrylbasis sowie Photoinitiatoren. Im ausgehärtetem Zustand sind in der Polyurethanmatrix die Schreibmonomere und die Photoinitiatoren räumlich verteilt eingebettet. Aus der WO-Schrift ist ebenfalls bekannt Dibutylphthalat, einen klassischen Weichmacher für technische Kunststoffe, der Photopolymer-Formulierung hinzuzufügen.

Für die Verwendungen von Photopolymer-Formulierungen in den unten beschriebenen Anwendungsfeldern spielt die durch die holographische Belichtung im Photopolymer erzeugte Brechungsindexmodulation $\Delta n$ die entscheidende Rolle. Bei der holographischen Belichtung wird das Interferenzfeld aus Signal- und Referenzlichtstrahl (im einfachsten Fall das zweier ebenen Wellen) durch die lokale Photopolymerisation von z.B. hochbrechenden Acrylate an Orten hoher Intensität im Interferenzfeld in ein Brechungsindexgitter abgebildet. Das Brechungsindexgitter im Photopolymeren (das Hologramm) enthält alle Information des Signallichtstrahls. Durch Beleuchtung des Hologramms nur mit dem Referenzlichtstrahl kann dann das Signal wieder rekonstruiert werden. Die Stärke des so rekonstruierten Signals im Verhältnis zur Stärke des eingestrahlten Referenzlichts wird Beugungseffizienz genannt, im folgenden DE wie Diffraction Efficiency. Im einfachsten Fall eines Hologramms, das aus der Überlagerung zweier ebener Wellen entsteht ergibt sich die DE aus dem Quotienten der Intensität des bei der Rekonstruktion abgebeugten Lichtes und der Summe der Intensitäten aus eingestrahltem Referenzlicht und abgebeugtem Licht. Je höher die DE desto effizienter ist ein Hologramm in Bezug auf die notwendige Lichtmenge des Referenzlichtes die notwendig ist um das Signal mit einer festen Helligkeit sichtbar zu machen. Hochbrechende Acrylate sind in der Lage, Brechungsindexgitter mit hoher Amplitude zwischen Bereichen mit niedrigstem Brechungsindex und Bereichen mit höchstem Brechungsindex zu erzeugen und damit in Photopolymer-Formulierungen Hologramme mit hohem DE und hohem $\Delta n$ zu ermöglichen. Dabei ist zu beachten das DE vom Produkt aus $\Delta n$ und der Photopolymerschichtdicke d abhängt. Je größer das Produkt desto größer die mögliche DE (für Reflexionshologramme). Die Breite des Winkelbereiches bei dem das Hologramm z.B. bei monochromatischer Beleuchtung sichtbar wird (rekonstruiert), hängt nur von der Schichtdicke d ab. Bei Beleuchtung des Hologramms mit z.B. weißem Licht hängt die Breite des spektralen Bereiches, der zur Rekonstruktion des Hologramms beitragen kann, ebenfalls nur von der Schichtdicke d ab. Dabei gilt: je kleiner d desto größer die jeweiligen Akzeptanzbreiten. Will man daher helle und leicht sichtbare Hologramme herstellen, ist ein hohes $\Delta n \cdot d$ und eine geringe Dicke d anzustreben und zwar so das DE möglichst groß wird. Das heißt je höher $\Delta n$ wird, desto mehr Freiraum zur Gestaltung heller Hologramme durch Anpassen von d und ohne Verlust an DE erreicht man. Daher kommt der Optimierung von $\Delta n$ bei der Optimierung von Photopolymerformulierungen eine herausragenden Bedeutung zu (Ref. Hariharan Optical Holography).

Eine Möglichkeit ein möglichst großes $\Delta n$ zu erreichen, ist die Verwendung von Photopolymer-Formulierungen, welche optisch niederbrechenden Weichmacher, also solche mit einem niedrigen Brechungsindex, enthalten. Neben den optischen Eigenschaften ist ein weiteres wichtiges Auswahlkriterium für solche Weichmacher, dass sie bei typischen Produktionsbedingungen für Photopolymer-Formulierungen hinreichend niederflüchtig sein müssen. Aufgrund eines beobachteten Masseverlust bei der Herstellung der holographischen Filmmedien liegt die Vermutung nahe, dass es während der Herstellung speziell im Bereich der Trocknung zur Verdampfung einzelner Komponenten kommen könnte. Bei der Betrachtung der Siedepunkte und der Dampfdrücke der eingesetzten Komponenten konnte jedoch nicht erwartet werden, dass diese bei den jeweiligen Trocknungstemperaturen eine Ursache für die Verdampfung und somit ein Grund für die deutlich niedrigeren $\Delta n$ Werte sein sollten.

Jedoch ist der Dampfdruck ein Parameter, mit dessen Hilfe die Eignung von Komponenten zur Verwendung bei der großtechnischen Herstellung von holographischen Medien nicht geprüft werden kann. Denn der Dampfdruck einer chemischen Verbindung ist eine Stoffkonstante, die beschreibt, wie ein Reinstoff oder ein Stoffgemisch im thermodynamischen Gleichgewicht mit seiner flüssigen oder festen Phase steht. Für dynamische Systeme liefert der Dampfdruck allerdings keine Anhaltpunkte.

So beschreibt der Dampfdruck nicht die Situation, wie sie zum Beispiel in einer kontinuierlich betriebenen Beschichtungsanlage herrscht, in der das mit einer geringen Schichtdicke applizierte Material verteilt über eine große Oberfläche durch eine Luftumwälzung, die dafür sorgt, dass die gasförmige Phase konstant abgeführt wird, getrocknet wird.

Zweckmäßigerweise wird stattdessen an Stelle des Dampfdrucks die Flüchtigkeit betrachtet. Da dieser Wert experimentell schwer zu bestimmen ist, könnten theoretische Methoden hierfür gut anwendbar sein.

Prinzipiell aus der Literatur bekannt sind Methoden bei denen, basierend auf einfachen molekularen Deskriptoren, Zusammenhänge zwischen den Molekül- und den Stoffeigenschaften einer Substanz, im Folgenden *Quantitative*

*Structure Property Relationships* (QSPR) genannt, gesucht werden. So werden beispielsweise von Ha et al. (Energy & Fuels, 19, 152, (2005)) QSPR-Modelle beschrieben, welche sich zur Abschätzung von Siedepunkten, relativen Dichten und Brechungsindices von gesättigten und aromatischen Kohlenwasserstoffen ohne Heteroatomen eignet. Von Liu *et al.* wurde ein QSPR Modell vorgestellt, welches sich vorrangig für Fluor und schwefelhaltige Kohlenwasserstoffe eignet (J. Micro/Nanolith. MEMS MOEMS 7, 023001-1-023001-11, (2008)). Diese Modelle werden auf Basis eines großen Satzes experimenteller Daten hergeleitet und erlauben häufig ein hohes Maß an Prädiktivität für Verbindungen die hinreichend ähnlich zu den zur Herleitung verwendeten Substanzen sind, weisen aber einer signifikant reduzierte Genauigkeit auf, wenn diese Ähnlichkeit nicht gegeben ist. Chandola et al. offenbaren ein computergestütztes Verfahren, das mit Hilfe von QSPR die Eignung von 25 Weichmachern für Polyvinylchlorid (PVC) berechnet. ("A QSPR for the plasticization efficiency of polyvinylchloride plasticizers", JOURNAL OF MOLECULAR GRAPHICS AND MODELLING, ELSEVIER SCIENCE, NEW YORK, NY, US, Bd. 26, Nr. 5, 6. Dezember 2007, Seiten 824-828, ISSN: 1093-3263).

Weitere bekannte Methode zur Abschätzung molekularer Eigenschaften sind Gruppelibeitragsmethoden, welche ein gegebenes Molekül in 'Gruppen' zerlegen, denen ein bestimmter inkrementeller Beitrag zu einer gegebenen physikalischen Eigenschaft zugewiesen wird. Die Stoffeigenschaften ergeben sich dann durch Aufsummieren der Gruppenbeiträge. Ein Beispiel ist die von 2001 von Marrero und Gani veröffentlichte Methode (Fluid Phase Equilib. 183-184, 183-208 (2001)). Ein prinzipielles Problem dieser Methoden stellt jedoch die Tatsache dar, dass keine Verbindungen behandelt werden können, deren Molekülstruktur von der Methode nicht erfasste Gruppen enthält. Darüberhinaus ist die Genauigkeit typischerweise für quantitative Aussagen nicht ausreichend.

In den WO 02/051263 und DE 10065443 sind QSPR Verfahren beschrieben, welche auf aus quantenchemischen Rechnungen abgeleiteten Deskriptoren beruhen. Die Methode wird dort verwendet, um das Phasenverhalten von Aromastoffen bzw. Riechstoffen in Multistoff-Systemen zu beschreiben. Diese Deskriptoren eignen sich besonders gut um einen vielseitigen Satz von Molekülen zu beschreiben, so dass QSPR-Modelle mit sehr wenigen Deskriptoren gefunden werden können, welche vielseitig einsetzbar sind.

Aufgabe der vorliegenden Erfindung war es, ein Auswahlverfahren für Additive in Photopolymer-Formulierungen bereitzustellen, die Herstellung von Hologrammen mit höherer Helligkeit ermöglichen.

Die Aufgabe wird bei der erfindungsgelnäßen Methode dadurch gelöst, dass der Brechungsindex und die Flüchtigkeit, ausgedrückt als TGA95 Wert, einer gegebenen noch nicht charakterisierten Substanz mit Hilfe eines mathematischen Verfahrens abgeschätzt wird, um deren Eignung als Additiv in Photopolymer-Formulierungen zu beurteilen. Eine Substanz gilt als geeignet, wenn ihr nach der erfindungsgemäßen Methode abgeschätzter Brechungsindex $\leq 1.4600$ sowie ihr abgeschätzter TGA95 Wert > 100°C ist.

Für die Beurteilung der Verdampfungseigenschaften einer Komponente mit einer niedrigen Schichtdicke während eines Trocknungsprozesses an offenen Oberflächen ist die Betrachtung der Flüchtigkeit dieser chemischen Komponente wichtig, die ein Maß für das Bestreben zum Verdunsten ist. Diese Flüchtigkeit kann vorzugsweise mit einer thermogravimetrischen Analyse (TGA) bestimmt werden.

Die TGA95 Werte der einzelnen Komponenten werden dann bestimmt, indem eine Menge von ca. 10 mg der Probe der jeweiligen Komponente in ein Aluminiumpfännchen mit einem Volumen von 70 $\mu$L eingewogen, das Aluminiumpfännchen in einen Ofen einer Thermowaage, bevorzugt einer Thermowaage TG50 der Firma Mettler-Toledo, eingebracht und bei einer konstanten Aufheizrate des Ofens von 20 K/min der Massenverlust der Probe in offenen Aluminiumpfännchen gemessen wird, wobei die Starttemperatur 30 °C und die Endtemperatur 600 °C des Ofens betragen, der Ofen während der Bestimmung mit einem. Stickstoffstrom einer Stärke von 200 ml/min durchgespült und als TGA 95 Wert der jeweiligen Komponente die Temperatur ermittelt wird, bei der ein Massenverlust der Probe von 5 Gew.-%, bezogen auf die ursprünglich eingewogene Menge der Probe, eingetreten ist.

Zur Anwendung des erfindungsgemäßen Auswahlverfahrens wird zunächst eine zu prüfende Substanz ausgewählt (Schritt a) von welcher mit Hilfe eines geeigneten Softwarepaketes, eine dreidimensionale Struktur erstellt wird. Diese Struktur wird dann, in an sich bekannter Weise, mit Hilfe eines Kraftfeldverfahrens, einer Konfonnerenanalyse unterzogen, um die energetisch niedrigsten Konformere der Substanz zu ermitteln (Schritt b). Die Anzahl der Konformere wird dann durch nochmaliges Geometrieoptimieren mit einem Kraftfeldverfahren und einer sich anschließenden Ähnlichkeitsanalyse, weiter reduziert (Schritt c). Die Schritte a-c können beispielsweise mit dem *conformers* Modul des Programmpaketes *Materials Studio* der Firma Accelrys durchgeführt werden.

Die in den Schritten a-c generierten Konformere werden dann quantenchemisch geometrieoptimiert, wobei idealerweise das B-P86 Dichtefunktional, sowie eine triple-$\zeta$-Valenzbasis verwendet werden (Schritt d). Die Geometrieoptimierung erfolgt unter Verwendung des Kontinuumsolvenzmodells *Conductor like Screening Model* (COSMO), wobei, soweit möglich, die von Klamt optimierten elementspezifischen COSMO-Radien verwendet werden (AlChE Journal, 48, 369, (2002); Fluid Phase Equilibria, 172, 43 (2000); J. Chem. Soc. Perkin Trans. II, 799 (1993)) . Sind Elemente in dem Molekül vorhanden, für welche keine solchen optimierten Radien vorhanden sind, so wird das 1.17-fache des Bondi-Valenzradius angenommen.

Auf Basis der COSMO-Radien wird von der verwendeten Quantenchemiesoftware eine Kavität berechnet außerhalb derer das Molekül während der Geometrieoptimierung ideal elektrostatisch gegen die Umgebung abgeschinnt

ist. Die Oberfläche (in Å$^2$) und das Volumen dieser Kavität (in Å$^3$) sind jeweils Deskriptoren im Rahmen des erfindungsmäßen Verfahrens (Schritt e).

Der dritte Deskriptor wird aus der Abschinnladungsoberfläche des geometrieoptimierten Moleküls abgeleitet, welche im Rahmen der quantenchemischen Geometrieoptimierung mit COSMO erhalten wird. Die Oberfläche wird mit Hilfe eines geeigneten Computerprogramms, wie zum Beispiel dem Programm COSMO*therm* der Finna COSMO*logic* in Segmente aufgeteilt, deren mittlere Abschirmladungsdichte (σ) berechnet wird. Der dritte Deskriptor wird dann letztendlich als zweites Moment (*M*$^2$) der Häufigkeitsverteilung (*P(σ)*), der Oberflächenabschirmladungsdichten dieser Segmente erhalten (Schritt f):

$$M^2 = 10 \cdot \sum_i P(\sigma_i) \cdot \sigma_i^2 \cdot \Delta\sigma ,$$

dabei ist Δσ die Intervallbreite mit der die diskrete Häufigkeitsverteilung generiert wurde. Die Ladungsdichten σ werden in der Einheit e/nm$^2$ angegeben.

Die auf die beschriebene Weise durchgeführte Berechnung der im erfindungsgemäßen Verfahren eingesetzten Deskriptoren *A*, *V* und *M*$^2$ erfolgt auf identische Weise für alle berücksichtigten Konformere. Nachfolgend werden die individuellen Deskriptoren der Konformere gemäß ihres Gewichtes in der Boltzmann-Verteilung, gemittelt (Schritt g). Die Boltzmann Faktoren werden dabei unter Verwendung der Energien ermittelt, die als Ergebnis der quantenchemischen Geometrieoptimierung mit COSMO erhalten wurden.

Mit den auf diese Weise ermittelten Deskriptoren wird abschließend der Brechungsindex und die Flüchtigkeit der zu prüfenden Substanz abgeschätzt. Zu diesem Zweck wird zunächst mit Hilfe eines aus der Literatur bekannten QSPR Ansatzes, beispielsweise mit einer der Methoden von Crippen et al. ( J. Comput. Chem., 7, 565, (1986) oder Chem. Inf. Comput. Sci. 39, 868, (1999)), die molare Polarisierbarkeit (MP) der zu prüfenden Verbindung ermittelt. Die Flüchtigkeit (TGA95) wird erfindungsgemäß entsprechend dem QSPR Ansatz:

$$TGA95 \approx 207.015 \cdot \frac{M^2}{A} + 41.405 \cdot \sqrt[3]{V} - 253.2$$

abgeschätzt (Schritt h). Die Dichte wird gemäß einem zweiten QSPR Ansatz erfindungsgemäß nach:

$$\rho = 0.89 \cdot \frac{M}{V \cdot N_A} - 0.2 \cdot \frac{A}{V} + 0.01 \cdot \sqrt{M^2}$$

abgeschätzt (Schritt i) und verwendet, um mit Hilfe der Lorentz-Lorenz Gleichung den Brechungsindex bei 589 nm ($n_D^{20}$) zu berechnen (Schritt j):

$$n_D = \sqrt{\frac{2 \cdot \frac{\rho \cdot MP}{M} + 1}{1 - \frac{\rho \cdot MP}{M}}}$$

Im letzten Schritt wird die Eignung der Substanz als Additiv in Photopolymer-Fonnulierungen entsprechend des abgeschätzten $n_D^{20}$ und TGA95 Wertes beurteilt (Schritt k).

Geeignete Verbindungen im Sinne des erfindungsgemäßen Verfahrens weisen einen Brechungsindex von ≤ 1.4600 sowie einen TGA95 Wert von ≥ 100°C auf.

**Bevorzugte Ausführungsformen**

Gemäß einer ersten Ausführungsform werden das energetisch niedrigste im Rahmen der Konformerenanalyse gefundene Konformer, bevorzugt alle Konformere welche bis zu 4kJ/mol und besonders bevorzugt alle Konformere welche bis zu 8 kJ/mol oberhalb des niedrigsten Konformers liegen, für das Verfahren berücksichtigt.

Bei einer besonders bevorzugten Ausführungsform des erfindungsgemäßen Verfahrens wird im Schritt k) geprüft, ob die Flüchtigkeit der zur prüfenden Verbindung > 120 °C und deren Brechungsindex $n_D^{20} \leq 1.4500$, bevorzugt ≤ 1.4400 besonders bevorzugt ≤1.4300 ist.

Ein weiterer Aspekt der Erfindung betrifft eine Photopolymer-Formulierung umfassend Matrixpolymere, Schreibmomomere und Photoinitiatoren, wobei sie wenigstens einen Weichmacher enthält, der nach dem erfindungsgemäßen Verfahren ausgewählt ist.

Bei den Matrixpolymeren kann es sich insbesondere um Polyurethane handeln. Vorzugsweise sind die Polyurethane durch Umsetzung einer Isocyanatkomponente a) mit einer Isocyanat-reaktiven Komponente b) erhältlich.

Die Isocyanatkomponente a) umfasst bevorzugt Polyisocyanate. Als Polyisocyanate können alle dem Fachmann an sich gut bekannten Verbindungen oder deren Mischungen eingesetzt werden, die im Mittel zwei oder mehr NCO-Funktionen pro Molekül aufweisen. Diese können auf aromatischer, araliphatischer, aliphatischer oder cycloaliphatischer Basis sein. In untergeordneten Mengen können auch Monoisocyanate und/oder ungesättigte Gruppen enthaltende Polyisocyanate mitverwendet werden.

Beispielsweise geeignet sind Butylendiisocyanat, Hexamethylendiisocyanat (HDI), Isophorondiisocyanat (IPDI), 1,8-Diisocyanato-4-(isocyanatomethyl)-octan, 2,2,4- und/oder 2,4,4-Trimethylhexainethylendiisocyanat, die isomeren Bis-(4,4'-isocyanatocyclohexyl)-methan und deren Mischungen beliebigen Isomerengehalts, Isocyanatomethyl-1,8-octandiisocyanat, 1,4-Cyaohexylendiisocyanat, die isomeren Cyclo-hexandimethylendiisocyanate, 1,4-Phenylendiisocyan a t , 2,4- und/oder 2,6-Toluylendiisocyanat, 1,5-Naphthylendiisocyanat, 2,4'- oder 4,4'-Diphenyhnethandiisocyanat und/oder Triphenylmethan-4,4',4''-triisocyanat.

Ebenfalls möglich ist der Einsatz von Derivaten monomerer Di- oder Triisocyanate mit Urethan-, Harnstoff-, Carbodiimid-, Acylharnstoff-, Isocyanurat-, Allophanat-, Biuret-, Oxadiazintrion-, Uretdion- und/oder Iminooxadiazindionstrukturen.

Bevorzugt ist der Einsatz von Polyisocyanaten auf Basis aliphatischer und/oder cycloaliphatischer Di- oder Triisocyanate.

Besonders bevorzugt handelt es sich bei den Polyisocyanaten der Komponente a) um di- oder oligomerisierte aliphatische und/oder cycloaliphatische Di- oder Triisocyanate.

Ganz besonders bevorzugt sind Isocyanurate, Uretdione und/oder Iminooxadiazindione basierend auf HDI, 1,8-Diisocyanato-4-(isocyanatomethyl)-octan oder deren Mischungen.

Ebenfalls können als Komponente a) NCO-funktionelle Prepolymere mit Urethan-, Allophanat-, Biuret- und/ oder Amidgruppen eingesetzt werden. Prepolymere der Komponente a) werden in dem Fachmann an sich gut bekannter Art und Weise durch Umsetzung von monomeren, oligomeren oder Polyisocyanaten a1) mit isocyanatreaktiven Verbindungen a2) in geeigneter Stöchiometrie unter optionalem Einsatz von Katalysatoren und Lösemitteln erhalten.

Als Polyisocyanate a1) eignen sich alle dem Fachmann an sich bekannten aliphatischen, cycloaliphatischen, aromatischen oder araliphatischen Di- und Triisocyanate, wobei es unerheblich ist, ob diese mittels Phosgenierung oder nach phosgenfreien Verfahren erhalten wurden. Daneben können auch die diem Fachmann an sich gut bekannten höhermolekularen Folgeprodukte monomerer Di- und/oder Triisocyanate mit Urethan-, Hamstoff-, Carbodiimid-, Acylharnstoff-, Isocyanurat-, Allophanat-, Biuret-, Oxadiazintrion-, Uretdion-, Iminooxadiazindionstruktur jeweils einzeln oder in beliebigen Mischungen untereinander eingesetzt werden.

Beispiele für geeignete monomere Di- oder Triisocyanate, die als Komponente a1) eingesetzt werden können, sind Butylendiisocyanat, Hexamethylendiisocyanat (HDI), Isophorondiisocyanat (IPDI), Trimethyl-hexamethylen-diisocyanat (TMDI), 1,8-Diisocyanato-4-(isocyanatomethyl)-octan, Isocyanatomethyl-1,8-octandiisocyanat (TIN), 2,4-und/ oder 2,6-Toluen-diisocyanat.

Als isocyanatreaktive Verbindungen a2) zum Aufbau der Prepolymere werden bevorzugt OHfunktionelle Verbindungen eingesetzt. Diese sind analog den OH-funktionellen Verbindungen wie sie nachfolgend für die Komponente b) beschrieben sind.

Ebenfalls möglich ist der Einsatz von Aminen zur Prepolymerherstellung. Beispielsweise geeignet sind Ethylendiamin, Diethylentriamin, Triethylentetramin, Propylendiamin, Diaminocyclohexan, Diaminobenzol, Diaminobisphenyl, difunktionelle Polyamine wie z.B. die Jeffamine®, amintenninierte Polymere mit zahlemnittleren Molmassen bis 10000 g/Mol oder deren beliebige Gemische untereinander.

Zur Herstellung von biuretgruppenhaltigen Prepolymeren wird Isocyanat im Überschuss mit Amin umgesetzt, wobei eine Biuretgruppe entsteht. Als Amine eignen sich in diesem Falle für die Umsetzung mit den erwähnten Di-, Tri- und Polyisocyanaten alle oligomeren oder polymeren, primären oder sekundären, difunktionellen Amine der vorstehend

genannten Art.

Bevorzugte Präpolymere sind Urethane, Allophanate oder Biurete aus aliphatischen Isocyanatfunktionellen Verbindungen und oligomeren oder polymeren Isocyanat-reaktiven Verbindungen mit zahlenmittleren Molmassen von 200 bis 10000 g/Mol, besonders bevorzugt sind Urethane, Allophanate oder Biurete aus aliphatischen Isocyanat-funktionellen Verbindungen und oligomeren oder polymeren Polyolen oder Polyaminen mit zahlenmittleren Molmassen von 500 bis 8500 g/Mol und ganz besonders bevorzugt sind Allophanate aus HDI oder TMDI und difunktionellen Polyetherpolyolen mit zahlenmittleren Molmassen von 1000 bis 8200 g/Mol.

Bevorzugt weisen die vorstehend beschriebenen Präpolymere Restgehalte an freiem monomeren Isocyanat von weniger als 1 Gew.-%, besonders bevorzugt weniger als 0.5 Gew.-%, ganz besonders bevorzugt weniger als 0.2 Gew.-% auf.

Selbstverständlich kann die Isocyanatkomponente anteilsmäßig neben den beschriebenen Präpolymeren weitere Isocyanatkomponenten enthalten. Hierfür kommen in Betracht aromatische, araliphatische, aliphatische und cycloaliphatische Di-, Tri- oder Polyisocyanate. Es können auch Mischungen solcher Di-, Tri- oder Polyisocyanate eingesetzt werden. Beispiele geeigneter Di-, Tri- oder Polyisocyanate sind Butylendiisocyanat, Hexamethylendiisoc yanat (HDI), I s o p horondiisocyanat (IPDI), 1,8-Diisocyanato-4-(isocyanatomethyl)octan, 2,2,4- und/oder 2,4,4-Trimethylhexamethylendiisocyanat (TMDI), die isomeren Bis(4,4'-isocyanatocyclohexyl)methane und deren Mischungen beliebigen Isomerengehalts, Isocyanatomethyl-1,8-octandiisocyanat, 1,4-Cyclohexylendiisocyanat, die isomeren Cyclohexandimethylendiisocyanate, 1,4-Phenylendiisocyanat, 2,4- und/oder 2,6-Toluylendiisocyanat, 1,5-Naphthylendiisocyanat, 2,4'- oder 4,4'-Diphenylmethandiisocyanat, Triphenylmethan-4,4',4"-triisocyanat oder deren Derivate mit Urethan-, Hamstoff-, Carbodiimid-, Acylharnstoff-, Isocyanurat-, Allophanat-, Biuret-, Oxadiazintrion-, Uretdion-, Iminooxadiazindionstruktur und Mischungen derselben. Bevorzugt sind Polyisocyanate auf Basis oligomerisierter und/oder derivatisierter Diisocyanate, die durch geeignete Verfahren von überschüssigem Diisocyanat befreit wurden, insbesondere die des Hexamethylendiiso-cyanat. Besonders bevorzugt sind die oligomeren Isocyanurate, Uretdione und Iminooxadiazindione des HDI sowie deren Mischungen.

Es ist gegebenenfalls auch möglich, dass die Isocyanatkomponente a) anteilsmäßig Isocyanate enthält, die teilweise mit isocyanat-reaktiven ethylenisch ungesättigten Verbindungen umgesetzt sind. Bevorzugt werden hierbei als isocyanat-reaktives ethylenisch ungesättigten Verbindungen a,β-ungesättigte Carbonsäurederivate wie Acrylate, Methacrylate, Maleinate, Fumarate, Maleimide, Acrylamide, sowie Vinylether, Propenylether, Allylether und Dicyclopentadienyl-Einheiten enthaltende Verbindungen, die mindestens eine gegenüber Isocyanaten reaktive Gruppe aufweisen, eingesetzt, besonders bevorzugt sind dies Acrylate und Methacrylate mit mindestens einer isocyanatreaktiven Gruppe. Als hydroxyfunktionelle Acrylate oder Methacrylate kommen beispielsweise Verbindungen wie 2-Hydroxy-ethyl(meth) acrylat, Polyethylenoxidanono(meth)acrylate, Polypropylenoxidmono(meth)acrylate, Polyalkylenoxidmono(meth)acrylate, Poly(E-caprolacton)mono(meth)acrylate, wie z.B. Tone® M100 (Dow, USA), 2-Hydroxypropyl(meth)acrylat, 4-Hydroxybutyl(meth)acrylat, 3-Hydroxy-2,2-dimethylpropyl(meth)acrylat, die hydroxyfunktionellen Mono-, D i- oder Tetra (meth)acrylate mehrwertiger Alkohole wie Trimethylolpropan, Glycerin, Pentaerythrit, Dipentaerythrit, ethoxyliertes, propoxyliertes oder alkoxyliertes Trimethylolpropan, Glycerin, Pentaerythrit, Dipentaerythrit oder deren technische Gemische in Betracht. Darüberhinaus sind isocyanat-reaktive oligomere oder polymere ungesättigte Acrylat- und/oder Methacrylatgruppen enthaltende Verbindungen alleine oder in Kombination mit den vorgenannten monomeren Verbindungen geeignet. Der Anteil an Isocyanaten die teilweise mit isocyanat-reaktiven ethylenisch ungesättigten Verbindungen umgesetzt sind an der Isocyanatkomponente a) beträgt 0 bis 99 %, bevorzugt 0 bis 50 %, besonders bevorzugt 0 bis 25 % und ganz besonders bevorzugt 0 bis 15 %.

Es ist gegebenenfalls auch möglich, dass die vorgenannten Isocyanatkomponente a) vollständig oder anteilsmäßig Isocyanate enthält, die ganz oder teilweise mit dem Fachmann aus der Beschichtungstechnologie bekannten Blockierungsimitteln umgesetzt sind. Als Beispiel für Blockierungsmittel seien genannt: Alkohole, Lactame, Oxime, Malonester, Alkylacetoacetate, Triazole, Phenole, Imidazole, Pyrazole sowie Amine, wie z.B. Butanonoxim, Diisopropylamin, 1,2,4-Triazol, Dimethyl-1,2,4-triazol, Imidazol, Malonsäurediethylester, Acetessigester, Acetonoxim, 3,5-Ditnethylpyrazol, ε-Caprolac.tam, N-tert.-Butyl-benzylainin, Cyclopentanoncarboxyethylester oder beliebige Gemische dieser Blockierungsmittel.

Als Komponente b) können an sich alle polyfunktionellen, isocyanatreaktiven Verbindungen eingesetzt werden, die im Mittel wenigstens 1.5 isocyanatreaktive-Gruppen pro Molekül aufweisen.

Isocyanatreaktive Gruppen im Rahmen der vorliegenden Erfindung sind bevorzugt Hydroxy-, Amino- oder Thiogruppen, besonders bevorzugt sind Hydroxyverbindungen.

Geeignete polyfunktionelle, isocyanatreaktive Verbindungen sind beispielsweise Polyester-, Polyether-, Polycarbonat-, Poly(meth)acrylat- und/oder Polyurethanpolyole.

Als Polyesterpolyole sind beispielsweise lineare Polyesterdiole oder verzweigte Polyesterpolyole geeignet, wie sie in bekannter Weise aus aliphatischen, cycloaliphatischen oder aromatischen Di- bzw. Polycarbonsäuren bzw. ihren Anhydriden mit mehrwertigen Alkoholen einer OH-Funktionalität ≥ 2 erhalten werden.

Beispiele für solche Di- bzw. Polycarbonsäuren bzw. Anhydride sind Bernstein-, Glutar-, Adipin-, Pimelin-,

Kork-, Azelain-, Sebacin-, Nonandicarbon-, Decandicarbon-, Terephthal-, Isophthal-, o-Phthal-, Tetrahydrophthal-, Hexahydrophthal- oder Trimellitsäure sowie Säureanhydride wie o-Phthal-, Trimellit- oder Bernsteinsäureanhydrid oder deren beliebige Gemische untereinander.

Beispiele für solche geeigneten Alkohole sind Ethandiol, Di-, Tri-, Tetraethylenglykol, 1,2-Propandiol, Di-, Tri-, Tetrapropylenglykol, 1,3-Propandiol, Butandiol-1,4, Butandiol-1,3, Butandiol-2,3, Pentandiol-1,5, Hexandiol-1,6, 2,2-Dimethyl-1,3-propandiol, 1,4-Dihydroxycyclohexan, 1,4-Dimethylolcyclohexan, Octandiol-1,8, Decandiol-1,10, Dodecandiol-1,12, Trimethylolpropan, Glycerin oder deren beliebige Gemische untereinander.

Die Polyesterpolyole können auch auf natürlichen Rohstoffen wie Rizinusöl basieren. Ebenfalls möglich ist, dass die Polyesterpolyole auf Homo- oder Mischpolymerisaten von Lactonen basieren, wie sie bevorzugt durch Anlagerung von Lactonen bzw. Lactongemischen wie Butyrolacton, ε-Caprolacton und/oder Methyl-ε-caprolacton an hydroxyfunktionelle Verbindungen wie mehrwertigen Alkohole einer OH-Funktionalität $\geq 2$ beispielsweise der vorstehend genannten Art erhalten werden können.

Solche Polyesterpolyole haben bevorzugt zahlenmittlere Molmassen von 400 bis 4000 g/Mol, besonders bevorzugt von 500 bis 2000 g/Mol. Ihre OH-Funktionalität beträgt bevorzugt 1.5 bis 3.5, besonders bevorzugt 1.8 bis 3.0.

Geeignete Polycarbonatpolyole sind in an sich bekannter Weise durch Umsetzung von organischen Carbonaten oder Phosgen mit Diolen oder Diol-Mischungen zugänglich.

Geeignete organische Carbonate sind Dimethyl-, Diethyl- und Diphenylcarbonat.

Geeignete Diole bzw. Mischungen umfassen die an sich im Rahmen der Polyestersegmente genannten mehrwertigen Alkoholen einer OH-Funktionalität $\geq 2$, bevorzugt 1,4-Butandiol, 1,6-Hexandiol und/oder 3-Methylpentandiol, oder auch Polyesterpolyole können zu Polycarbonatpolyolen umgearbeitet werden.

Solche Polycarbonatpolyole haben bevorzugt zahlenmittlere Molmassen von 400 bis 4000 g/Mol, besonders bevorzugt von 500 bis 2000 g/Mol. Die OH-Funktionalität dieser Polyole beträgt bevorzugt 1.8 bis 3.2, besonders bevorzugt 1.9 bis 3.0.

Geeignete Polyetherpolyole sind gegebenenfalls blockweise aufgebaute Polyadditionsprodukte cyclischer Ether an OH- oder NH-funktionelle Startennoleküle.

Geeignete cyclische Ether sind beispielsweise Styroloxide, Ethylenoxid, Propylenoxid, Tetrahydrofuran, Butylenoxid, Epichlorhydrin, sowie ihre beliebigen Mischungen.

Als Starter können die an sich im Rahmen der Polyesterpolyole genannten mehrwertigen Alkohole einer OH-Funktionalität $\geq 2$ sowie primäre oder sekundäre Amine und Aminoalkohole verwendet werden.

Bevorzugte Polyetherpolyole sind solche der vorgenannten Art ausschließlich basierend auf Propylenoxid oder statistische oder Block-Copolymere basierend auf Propylenoxid mit weiteren 1-Alkylenoxiden, wobei der 1-Alykenoxidanteil nicht höher als 80 Gew.-% ist. Daneben sind Poly(trimethylenoxide) sowie Mischungen der als bevorzugt genannten Polyole bevorzugt. Besonders bevorzugt sind Propylenoxid-homopolymere sowie statistische oder Block-Copolymere, die Oxyethylen-, Oxypropylen- und/oder Oxybutyleneinheiten aufweisen, wobei der Anteil der Oxypropyleneinheiten bezogen auf die Gesamtmenge aller Oxyethylen-, Oxypropylen- und Oxybutyleneinheiten mindestens 20 Gew.-%, bevorzugt mindestens 45 Gew.-% ausmacht. Oxypropylen- und Oxybutylen- umfasst hierbei alle jeweiligen linearen und verzweigten C3- und C4-Isomere.

Solche Polyetherpolyole haben bevorzugt zahlenmittlere Molmassen von 250 bis 10000 g/Mol, besonders bevorzugt von 500 bis 8500 g/Mol und ganz besonders bevorzugt von 600 bis 4500 g/Mol. Die OH-Funktionalität beträgt bevorzugt 1.5 bis 4.0, besonders bevorzugt 1.8 bis 3.1.

Daneben sind als Bestandteile der Komponente c) als polyfunktionelle, isocyanatreaktive Verbindungen auch niedermolekulare, d.h. mit Molekulargewichten kleiner 500 g/mol, kurzkettige, d.h. 2 bis 20 Kohlenstoffatome enthaltende aliphatische, araliphatische oder cycloaliphatische di, tri oder polyfunktionelle Alkohole geeignet.

Dies können beispielsweise sein Ethylenglykol, Diethylenglykol, Triethylenglykol, Tetraethylenglykol, Dipropylenglykol, Tripropylenglykol, 1,2-Propandiol, 1,3-Propandiol, 1,4-butandiol, Neopentylglykol, 2-Ethyl-2-butylpropandiol, Trimethylpentandiol, stellungsisomere Diethyloctandiole, 1 ,3-Butylenglykol, Cyclohexandiol, 1,4-Cyclohexandimethanol, 1,6-Hexandiol, 1,2- und 1,4-Cyclohexandiol, hydriertes Bisphenol A (2,2-Bis(4-hydroxycyclo-hexyl)propan), 2,2-Dimethyl-3-hydroxypropionsäure (2,2-dimethyl-3-hydroxypropylester). Beispiele geeigneter Triole sind Trimethylolethan, Trimethylolpropan oder Glycerin. Geeignete höherfunktionelle Alkohole sind Ditrimethylolpropan, Pentaerythrit, Dipentaerythrit oder Sorbit.

Als Komponente c) werden ein oder mehrere Photoinitiatoren eingesetzt. Dies sind üblicherweise durch aktinische Strahlung aktivierbare Initiatoren, die eine Polymerisation der entsprechenden polymerisierbaren Gruppen auslösen. Photoinitiatoren sind an sich bekannte, kommerziell vertriebene Verbindungen, wobei zwischen unimolekularen (Typ I) und bimolekularen (Typ II) Initiatoren unterschieden wird. Desweiteren werden diese Initiatoren je nach chemischer Natur für die radikalische, die anionische (oder), die kationische (oder gemischte) Formen der vorgenannten Polymerisationen eingesetzt.

(Typ I)-Systeme für die radikalische Photopolymerisation sind z.B. aromatische Ketonverbindungen, z.B. Benzophenone in Kombination mit tertiären Aminen, Alkylbenzophenone, 4,4'-Bis(dimethylamino)benzophenon (Michlers

Keton), Anthron und halogenierte Benzophenone oder Mischungen der genannten Typen. Weiter geeignet sind (Typ II)-Initiatoren wie Benzoin und seine Derivate, Benzilketale, Acylphosphinoxide z.B. 2,4,6-Trimethyl-benzoyl-diphenylphosphinoxid, Bisacylophosphinoxide, Phenylglyoxylsäureester, Campherchinon, alpha-Aminoalkylphenone, alpha-,alpha-Dialkoxyacetophenone, 1-[4-(Phenylthio)phenyl]octan-1,2-dion-2-(O-be,zoyloxim), u n terschiedlich substituierte Hexarylbisimidazole (HABI) mit geeigneten Coinitiatoren wie z.B. Mercaptobenzoxazol sowie alpha-Hydroxyalkylphenone. Auch die in EP-A 0223587 beschriebenen Photoinitiatorsysteme bestehend aus einer Mischung aus einem Ammoniumarylborat und einem oder mehreren Farbstoffen können als Photoinitiator eingesetzt werden. Als Ammoniumarylborat eignen sich beispielsweise Tetrabutylammonium Triphenylhexylborat, Tetrabutylammonium Triphenylbutylborat, Tetrabutylammonium Trinaphthylbutylborat, Tetramethylammonium Triphenylbenzylborat, Tetra(n-hexyl)ammonium (sec-Butyl)triphenylborat, 1-Methyl-3-octylimidazolium Dipentyldiphenylborat, Tetrabutylammonium Tris-(4-tert.-butyl)-phenylbutylborat, Tetrabutylammonium Tris-(3-fluorphenyl)-hexylborat und Tetrabutylammonium Tris-(3-Chlor-4-methylphenyl)-hexylborat. Als Farbstoffe eignen sich beispielsweise Neu-Methylenblau, Thionin, Basic Yellow, Pinacynol Chlorid, Rhodamin 6G, Gallocyanin, Ethylviolett, Victoria Blue R, Celestine Blue, Chinaldinrot, Kristallviolett, Brilliant Grün, Astrazon Orange G, Darrow Red, Pyronin Y, Basic Red 29, Pyrillium I, Safranin O, Cyanin und Methylenblau, Azur A (Cunningham et al., RadTech'98 North America UV/EB Conference Proceedings, Chicago, Apr. 19-22, 1998).

Die für die anionische Polymerisation verwendeten Photoinitiatoren sind in der Regel (Typ I)-Systeme und leiten sich von Übergangsmetall-Komplexen der ersten Reihe ab. Hier sind Chrom-Salze, wie z.B. trans-$Cr(NH_3)_2(NCS)_4$- (Kutal et al, Macromolecules 1991, 24, 6872) oder Ferrocenyl-Verbindungen (Yamaguchi et al., Macromolecules 2000, 33, 1152). Eine weitere Möglichkeit der anionischen Polymerisation besteht in der Verwendung von Farbstoffen, wie Kristallviolett Leukonitril oder Malchit Grün Leukonitril, die durch photolytischen Zerfall Cyanoacrylate polymerisieren können (Neckers et al., Macromolecules 2000, 33, 7761). Allerdings wird dabei das Chromophor in das Polymer eingebaut, so dass die resultierenden Polymere durchgefärbt sind.

Die für die kationische Polymerisation verwendeten Photoinitiatoren bestehen im wesentlichen aus drei Klassen: Aryldiazonium-Salze, Onium-Salze (hier speziell: Iodonium-, Sulfonium-und Selenonium-Salze) sowie Organometall-Verbindungen. Phenyldiazonium-Salze können unter Bestrahlung sowohl in Gegenwart als auch in Abwesenheit eines Wasserstoff-Donors ein Kation erzeugten, dass die Polymerisation initiiert. Die Effizienz des Gesamtsystems wird durch die Natur des verwendeten Gegenions zur Diazonium-Verbindung bestimmt. Bevorzugt sind hier die wenig reaktiven aber recht teuren $SbF_6^-$, $AsF_6^-$ oder $PF_6^-$. Für den Einsatz in Beschichtung dünner Filme sind diese Verbindungen i.d.R wenig geeignet, da durch den nach der Belichtung freigesetzten Stickstoff die Oberflächegüte herabgesetzt wird (pinholes) (Li et al., Polymeric Materials Science and Engineering, 2001, 84, 139). Sehr weit verbreitet und auch in vielerlei Form kommerziell erhältlich sind Onium-Salze, speziell Sulfonium- und Iodonium-Salze. Die Photochemie dieser Verbindungen ist nachhaltig untersucht worden. Die Iodonium-Salze zerfallen nach der Anregung zunächst homolytisch und erzeugen somit ein Radikal und ein Radikalanion, welches sich durch H-Abstraktion stabilisiert und ein Proton freisetzt und dann die kationische Polymerisation startet (Dektar et al. J. Org. Chem. 1990, 55, 639; J. Org. Chem., 1991, 56, 1838). Dieser Mechanimus ermöglicht den Einsatz von Iodonium-Salzen ebenfalls für die radikalische Photopolymerisation. Hierbei kommt erneut der Wahl des Gegenions eine große Bedeutung zu, bevorzugt werden ebenfalls $SbF_6^-$, $AsF_6^-$ oder $PF_6^-$. Ansonsten in dieser Strukturklasse die Wahl der Substitution des Aromaten recht frei und im wesentlichen durch die Verfügbarkeit geeigneter Startbausteine für die Synthese bestimmt. Bei den Sulfonium-Salzen handelt es sich um Verbindungen, die in nach Norrish(II) zerfallen (Grivello et al., Macromolecules, 2000, 33, 825). Auch bei den Sulfonium-Salzen kommt der Wahl des Gegenions eine kritische Bedeutung zu, die sich im Wesentlichen in der Härtungsgeschwindigkeit der Polymere äußert. Die besten Ergebnisse werden i.d.R. mit $SbF_6$Salzen erzielt. Da die Eigenabsorption von Iodonium- und Sulfonium-Salze bei <300nm liegt, müssen diese Verbindungen für die Photopolymerisation mit nahem UV oder kurzwelligem sichtbarem Licht entsprechend sensibilisiert werden. Dies gelingt durch die Verwendung von höher absorbierenden Aromaten wie z.B. Anthracen und Derivaten (Gu et al., Am. Chem. Soc. Polymer Preprints, 2000, 41 (2), 1266) oder Phenothiazin bzw. dessen Derivate (Hua et al, Macromolecules 2001, 34, 2488-2494).

Es kann vorteilhaft sein auch Gemische dieser Verbindungen einzusetzen. Je nach zur Härtung verwendeter Strahlungsquelle muss Typ und Konzentration an Photoinitiator in dem Fachmann bekannter Weise angepasst werden. Näheres ist zum Beispiel in P. K. T. Oldring (Ed.), Chemistry & Technology of UV & EB Formulations For Coatings, Inks & Paints, Vol. 3, 1991, SITA Technology, London, S. 61 - 328 beschrieben.

Bevorzugte Photoinitiatoren c) sind Mischungen aus Tetrabutylammonium Triphenylhexylborat, Tetrabutylammonium Triphenylbutylborat, Tetrabutylammonium Trinapthylbutylborat, Tetrabutylammonium Tris-(4-tert.-butyl)-phenylbutylborat, Tetrabutylammonium Tris-(3-fluorphenyl)-hexylborat und Tetrabutylammonium Tris-(3-Chlor-4-methylphenyl)-hexylborat mit Farbstoffen wie beispielsweise Astrazon Orange G, Methylenblau, Neu Methylenblau, Azur A, Pyrillium I, Safranin O, Cyanin, Gallocyanin, Brilliant Grün, Kristallviolett, Ethylviolett und Thionin.

Die Photopolymer-Formulierung kann zusätzlich Urethane als Weichmacher enthalten, wobei die Urethane bevorzugt mit wenigstens einem Fluoratom substituiert sein können.

Bei den Urethanen handelt es sich vorzugsweise um Verbindungen, die ein Strukturelement der allgemeinen Formel I aufweisen.

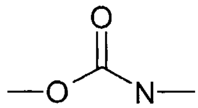
Formel I

Sie können aus monofunktionellen Alkoholen und monofunktionellen Isocyanaten erhalten werden. Bevorzugt sind diese mit wenigstens einem Fluoratom substituiert.

Weiter bevorzugt ist, wenn die Fluorurethane die allgemeine Formel II

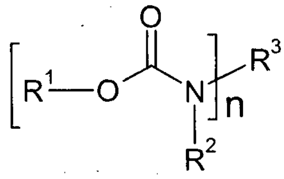
Formel II

aufweisen, in der n≥1 und n≤8 ist und $R^1$, $R^2$, $R^3$ Wasserstoff und / oder unabhängig voneinander lineare, verzweigte, cyclische oder heterocyclische unsubstituierte oder gegebenenfalls auch mit Heteroatomen substituierte organische Reste sind, wobei mindestens einer der Reste $R^1$, $R^2$, $R^3$ mit wenigstens einem Fluoratom substituiert ist. Besonders bevorzugt ist hierbei $R^1$ ein organischer Rest mit mindestens einem Fluoratom.

Gemäß einer weiteren Ausführungsform kann $R^1$ 1-20 $CF_2$ Gruppen und / oder eine oder mehrere $CF_3$ Gruppen, besonders bevorzugt 1-15 $CF_2$ Gruppen und / oder eine oder mehrere $CF_3$ Gruppen, insbesondere bevorzugt 1-10 $CF_2$ Gruppen und / oder eine oder mehrere $CF_3$ Gruppen, ganz besonders bevorzugt 1-8 $CF_2$ Gruppen und / oder eine oder mehrere $CF_3$ Gruppen, $R^2$ einen C1-C20 Alkyl-Rest, bevorzugt einen C1-C15 Alkyl-Rest besonders bevorzugt einen C1-C10 Alkyl-Rest oder Wasserstoff, und / oder $R^3$ einen C1-C20 Alkyl-Rest, bevorzugt einen C1-C15 Alkyl-Rest, besonders bevorzugt einen C1-C10 Alkyl-Rest oder Wasserstoff umfassen.

Die Fluorurethane können einen Fluorgehalt von 10-80 Gew.-% Fluor, bevorzugt von 13-70 Gew.-% Fluor und besönders bevorzugt 17.5-65 Gew.-% Fluor aufweisen.

Gemäß einer weiteren bevorzugten Ausführungsform der Erfindung ist vorgesehen, dass die Photopolymer-Formulierung 10 bis 89.999 Gew.-%, bevorzugt 25 bis 70 Gew.-% Matrixpolymere, 10 bis 60 Gew.-%, bevorzugt 25 bis 50 Gew.-% Schreibmonomere, 0.001 bis 5 Gew.-% Photoinitiatoren und gegebenenfalls 0 bis 4 Gew.-%, bevorzugt 0 bis 2 Gew.-% Katalysatoren, 0 bis 5 Gew.-% , bevorzugt 0.001 bis 1 Gew.-% Radikalstabilisatoren 0 bis 30 Gew.-%, bevorzugt 0 bis 25 Gew.-% Weichmacher und 0 bis 5 Gew.-%, bevorzugt 0.1 bis 5 Gew.-% weitere Additive enthält, wobei die Summe aller Bestandteile 100 Gew.-% beträgt.

Besonders bevorzugt werden Photopolymer-Formulierungen mit 25 bis 70 Gew.-% Matrixpolymeren bestehend aus Verbindungen der Komponente a) und der Komponente b), 25 bis 50 Gew.-% Schreibmonomere, 0.001 bis 5 Gew.-% Photoinitiatoren, 0 bis 2 Gew.-% Katalysatoren, 0,001 bis 1 Gew.-% Radikalstabilisatoren gegebenenfalls 0 bis 25 Gew.-% der oben beschriebenen Urethane und gegebenenfalls 0.1 bis 5 Gew.-% weiterer Additive eingesetzt.

Eine weitere bevorzugte Ausführungsform der Erfindung sieht vor, dass die Photopolymerformulierung Urethane mit einem zahlenmittleren Molekulargewicht von ≤ 250 g/mol, bevorzugt von ≤ 200 g/mol und insbesondere bevorzugt von ≤ 190 g/mol enthält.

Vorteilhafterweise enthält die Photopolymer-Formulierung daher aliphatische Urethane. In diesem Fall können die aliphatischen Urethane insbesondere die die allgemeine Formel (III)

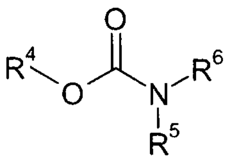
(III)

aufweisen, in der $R^4$, $R^5$, $R^6$ unabgängig voneinander lineare oder verzweigte, gegebenenfalls mit Heteroatomen substituierte, (C1-C20)-Alkyl-Reste sind. Besonders bevorzugt ist, dass $R^4$ lineare oder verzweigte (C1-C8)-Alkyl-Reste, $R^5$ lineare oder verzweigte (C1-C8)-AlkylReste, und / oder $R^6$ lineare oder verzweigte (C1-C8)-Alkyl-Reste, sind, wobei

insbesondere bevorzugt ist, wenn $R^4$ lineare oder verzweigte (C1-C4)-Alkyl-Reste, ist und $R^5$ lineare oder verzweigte (C1-C6)-Alkyl-Reste, ist und $R^6$ Wasserstoff ist. Es wurde nämlich in diesem Fall festgestellt, dass die so erhaltenen Urethane sehr gut mit der Polyurethan-Matrix verträglich sind und den hier beschriebenen Effekt zeigen.

Gemäß einer weiteren bevorzugten Ausführungsform ist vorgesehen, dass die Urethane im Wesentlichen keine freien NCO-Gruppen aufweisen.

Vorzugsweise können die Schreibmonomere ein monofunktionelles Acrylat der allgemeinen Formel (IV)

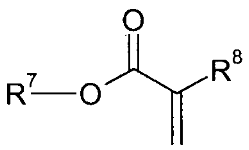

(IV)

umfassen, in der $R^7$, $R^8$ Wasserstoff und / oder unabhängig voneinander lineare, verzweigte, cyclische oder heterocyclische unsubstituierte organische Reste sind.

Es ist ebenfalls möglich, dass die Schreibmonomere ein multifunktionales Schreibmonomerumfassen, wobei es sich insbesondere um ein multifunktionelles Acrylat handeln kann.

Das multifunktionelle Acrylat kann bevorzugt die allgemeine Formel (V)

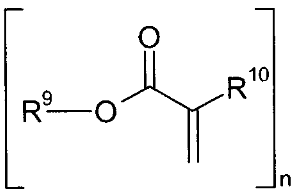

(V)

haben, in der n≥2 und n≤4 ist und $R^9$, $R^{10}$ Wasserstoff und / oder unabhängig voneinander lineare, verzweigte, cyclische oder heterocyclische unsubstituierte oder gegebenenfalls auch mit Heteroatomen substituierte organische Reste sind.

Weiterer Gegenstand der vorliegenden Erfindung ist die Verwendung einer erfindungsgemäßen Photopolymer-Formulierung zur Herstellung holographischer Medien, insbesondere zur Herstellung von In-Line Hologrammen, Off-Axis Hologrammen, Full-Aperture Transfer Hologrammen, Weißlicht-Transmissionshologrammen, Denisyukhologrammen, Off-Axis Reflektionshologrammen, Edge-Lit Hologrammen sowie holographischen Stereogrammen.

## Beispiele

Die Erfindung wird im Folgenden anhand von Beispielen näher erläutert. Dabei wird zunächst die Synthese und Charakterisierung der Beispielmoleküle beschrieben und im Anschluss deren Behandlung im Rahmen des erfindungsgemäßen Verfahrens.

Sofern nicht abweichend vermerkt beziehen sich alle Prozentangaben auf Gewichtsprozent.

## Bestimmung des Brechungsindex

Messung des Brechungsindex $n_D^{20}$ bei einer Wellenlänge von 589 nm: Eine Probe der Beispiel-Verbindung wurde in ein Abbe-Refraktometer gegeben und $n_D^{20}$ gemessen.

## Bestimmung des Dampfdrucks

Für die Beispiele 2 und 7, deren Synthese weiter unten beschrieben wird, wurden die Dampfdrücke unter Stickstoffatmosphäre in einer Umlaufapparatur (isobar in einem Ebulliometer) nach Röck gemäß der OECD Richtlinie zum Testen von Chemikalien Nr. 104 bestimmt. Die resultierenden Dampfdruckkurven sind im Temperaturbereich von 90 bis 180 °C in Figur 5 abgebildet. Die resultierenden Parameter der Antoine-Gleichung

$$\lg \frac{P^{Sat}}{hPa} = A - \frac{B}{C + T(°C)}$$

sind danach:

Tabelle 1: Stoffdaten der Beispiele **2** und **7**

| Beispiel | Parameter der Antoine-Gleichung | | | Dampfdruck | Dampfdruck | TGA 95 |
|---|---|---|---|---|---|---|
| | | | | bei 80 °C | bei 100 °C | [°C] |
| | | | | [hPa] | [hPa] | |
| | A | B | C | | | |
| **2** | 15.6189 | 2616.075 | 90.3596 | 1.30 | 7.30 | 72.5 |
| **7** | 6.5466 | 1053.404 | 42.458 | 0.13 | 0.42 | 111.8 |

Hieraus ist zu sehen, dass der Gleichgewichtsdampfdruck für beispielsweise eine Temperatur von 100 °C für beide Beispiele **2** und **7** < 10 hPa ist, so dass man erwarten würde, dass die beiden Komponenten eine ausreichende Beständigkeit in der Formulierung haben. Die TGA95 Werte der beiden Beispiele **2** und **7** dagegen korrelieren deutlich besser mit dem beobachteten Verhalten während einer Folienbeschichtung.

Figur 1 zeigt die gemessenen Dampfdruckkurven der Beispiele **2** und **7.**

**<u>Bestimmung des TGA95-Wertes</u>**

Die TGA95 Werte der einzelnen Komponenten werden bestimmt, indem eine Menge von ca. 10 mg der jeweiligen Komponente in ein Aluminiumpfännchen mit einem Volumen von 70 μl eingewogen, das Aluminiumpfännchen in einen Ofen einer Thermowaage, bevorzugt einer Thermowaage TG50 der Firma Mettler-Toledo, eingebracht und bei einer konstanten Aufheizrate des Ofens von 20 K/min der Massenverlust der Probe im offenen Aluminiumpfännchen gemessen wird, wobei die Starttemperatur 30 °C und die Endtemperatur 600 °C des Ofens betragen, der Ofen während der Bestimmung mit einem Stickstoffstrom einer Stärke von 200 ml/min durchgespült und als TGA95 Wert der jeweiligen Komponente die Temperatur ermittelt wird, bei der ein Massenverlust der Probe von 5 Gew.-%, bezogen auf die ursprünglich eingewogene Menge der Probe, eingetreten ist.

**<u>Messung der holographischen Eigenschaften DE und Δn der holographischen Medien mittels Zweistrahlinterferenz in Reflexionsanordnung</u>**

Zur Messung der holographischen Perfonnance wird die Schutzfolie des holographischen Films abgezogen und der holographische Film mit der Photopolymerseite auf eine 1 mm dicke Glasplatte geeigneter Länge und Breite mit einer Gummiwalze unter leichtem Druck auflaminiert. Dieser Sandwich aus Glas und Photopolymerfolie kann nun verwendet werden, um die holographischen Performanceparameter DE und Δn zu bestimmen.

Die wie unten beschrieben hergestellten holographischen Medien wurden anschließend mittels einer Messanordnung gemäß Figur 3 wie folgt auf ihre holographischen Eigenschaften geprüft:

Der Strahl eines He-Ne Lasers (Emissionswellenlänge 633 nm) wurde mit Hilfe des Raumfilter (SF) und zusammen mit der Kollimationslinse (CL) in einen parallelen homogenen Strahl umgewandelt. Die finalen Querschnitte des Signal und Referenzstrahls werden durch die Irisblenden (I) festgelegt. Der Durchmesser der Irisblendenöffnung beträgt 0.4 cm. Die polarisationsabhängigen Strahlteiler (PBS) teilen den Laserstrahl in zwei kohärente gleich polarisierte Strahlen. Über die λ/2 Plättchen wurden die Leistung des Referenzstrahls auf 0.5 mW und die Leistung des Signalstrahls auf 0.65 mW eingestellt. Die Leistungen wurden mit den Halbleiterdetektoren (D) bei ausgebauter Probe bestimmt. Der Einfallswinkel ($\alpha_0$) des Referenzstrahls beträgt -21.8°, der Einfallswinkel ($\beta_0$) des Signalstrahls beträgt 41.8°. Die Winkel werden ausgehend von der Probennormale zur Strahlrichtung gemessen. Gemäß Figur 3 hat daher $\alpha_0$ ein negatives Vorzeichen und $\beta_0$ ein positives Vorzeichen. Am Ort der Probe (Medium) erzeugte das Interferenzfeld der zwei überlappenden Strahlen ein Gitter heller und dunkler Streifen die senkrecht zur Winkelhalbierenden der zwei auf die Probe einfallenden. Strahlen liegen (Reflexionshologramm). Der Streifenabstand Λ, auch Gitterperiode genannt, im Medium beträgt ~ 225 nm (der Brechungsindex des Mediums zu ~1.504 angenommen).

Figur 3 zeigt den holographischen Versuchsaufbau, mit dem die Beugungseffizienz (DE) der Medien gemessen wurde.

Es wurden auf folgende Weise Hologramme in das Medium geschrieben:

- Beide Shutter (S) sind für die Belichtungszeit *t* geöffnet.
- Danach wurde bei geschlossenen Shuttern (S) dem Medium 5 Minuten Zeit für die Diffusion der noch nicht polymerisierten Schreibmonomere gelassen.

Die geschriebenen Hologramme wurden nun auf folgende Weise ausgelesen. Der Shutter des Signalstrahls blieb geschlossen. Der Shutter des Referenzstrahls war geöffnet. Die Irisblende des Referenzstrahls wurde auf einen Durchmesser < 1 mm geschlossen. Damit erreichte man, dass für alle Drehwinkel ($\Omega$) des Mediums der Strahl immer vollständig im zuvor geschriebenen Hologramm lag. Der Drehtisch überstrich nun computergesteuert den Winkelbereich von $\Omega_{min}$ bis $\Omega_{max}$ mit einer Winkelschrittweite von 0.05°. $\Omega$ wird von der Probennormale zur Referenzrichtung des Drehtisches gemessen. Die Referenzrichtung des Drehtisches ergibt sich dann wenn beim Schreiben des Hologramms der Einfallswinkel des Referenz- und des Signalstrahls betragsmäßig gleich sind also $\alpha_0$ = -31.8° und $\beta_0$ = 31.8° gilt. Dann beträgt $\Omega_{recording}$ = 0°. Für $\alpha_0$ = -21.8° und $\beta_0$ = 41.8° beträgt $\Omega_{recording}$ daher 10°. Allgemein gilt für das Interferenzfeld beim Schreiben ("recording") des Hologramms:

$$\alpha_0 = \theta_0 + \Omega_{recording}.$$

$\theta_0$ ist der Halbwinkel im Laborsystem außerhalb des Mediums und es gilt beim Schreiben des Hologramms:

$$\theta_0 = \frac{\alpha_0 - \beta_0}{2}.$$

In diesem Fall gilt also $\theta_0$ = -31.8°. An jedem angefahrenen Drehwinkel $\Omega$ wurden die Leistungen des in der nullten Ordnung transmittierten Strahls mittels des entsprechenden Detektors D und die Leistungen des in die erste Ordnung abgebeugten Strahls mittels des Detektors D gemessen. Die Beugungseffizienz ergab sich bei jedem angefahrenen Winkel $\Omega$ als der Quotient aus:

$$\eta = \frac{P_D}{P_D + P_T}$$

$P_D$ ist die Leistung im Detektor des abgebeugten Strahls und $P_T$ ist die Leistung im Detektor des transmittierten Strahls.

Mittels des oben beschriebenen Verfahrens wurde die Braggkurve, sie beschreibt den Beugungswirkungsgrad $\eta$ in Abhängigkeit des Drehwinkels $\Omega$ des geschriebenen Hologramms gemessen und in einem Computer gespeichert. Zusätzlich wurde auch die in die nullte Ordnung transmittierte Intensität gegen den Drehwinkel $\Omega$ aufgezeichnet und in einem Computer gespeichert.

Die maximale Beugungseffizienz (DE = $\eta_{max}$) des Hologramms, also sein Spitzenwert, wurde bei $\Omega_{reconstruction}$ ermittelt. Eventuell musste dazu die Position des Detektors des abgebeugten Strahls verändert werden, um diesen maximalen Wert zu bestimmen.

Der Brechungsindexkontrast $\Delta n$ und die Dicke d der Photopolymerschicht wurde nun mittels der Coupled Wave Theorie (siehe; H. Kogelnik, The Bell System Technical Journal, Volume 48, November 1969, Number 9 Seite 2909 - Seite 2947) an die gemessene Braggkurve und den Winkelverlauf der transmittierten Intensität ermittelt. Dabei ist zu beachten, dass wegen der durch die Photopolymerisation auftretenden Dickenschwindung der Streifenabstand $\Lambda'$ des Hologramms und die Orientierung der Streifen (slant) vom Streifenabstand $\Lambda$ des Interferenzmusters und dessen Orientierung abweichen kann. Demnach wird auch der Winkel $\alpha_0'$ bzw. der entsprechende Winkel des Drehtisches $\Omega_{reconstruction}$, bei dem maximale Beugungseffizienz erreicht wird von $\alpha_0$ bzw. vom entsprechenden $\Omega_{recording}$ abweichen. Dadurch verändert sich die Bragg-Bedingung. Diese Veränderung wird im Auswerteverfahren berücksichtigt. Das Auswerteverfahren wird im Folgenden beschrieben:

Alle geometrischen Größen, die sich auf das geschriebene Hologramm beziehen und nicht auf das Interferenzmuster werden als gestrichene Größen dargestellt.

Für die Braggkurve η(Ω) eines Reflexionshologramms gilt nach Kogelnik:

$$\eta = \begin{cases} \dfrac{1}{1 - \dfrac{1-(\xi/\nu)^2}{\sin^2\left(\sqrt{\xi^2-\nu^2}\right)}}, \text{ für } \nu^2 - \xi^2 < 0 \\ \dfrac{1}{1 + \dfrac{1-(\xi/\nu)^2}{\sinh^2\left(\sqrt{\nu^2-\xi^2}\right)}}, \text{ für } \nu^2 - \xi^2 \geq 0 \end{cases}$$

mit:

$$\nu = \frac{\pi \cdot \Delta n \cdot d'}{\lambda \cdot \sqrt{|c_s \cdot c_r|}}$$

$$\xi = -\frac{d'}{2 \cdot c_s} \cdot DP$$

$$c_s = \cos(\vartheta') - \cos(\psi') \cdot \frac{\lambda}{n \cdot \Lambda'}$$

$$c_r = \cos(\vartheta')$$

$$DP = \frac{\pi}{\Lambda'} \cdot \left(2 \cdot \cos(\psi' - \vartheta') - \frac{\lambda}{n \cdot \Lambda'}\right)$$

$$\psi' = \frac{\beta' + \alpha'}{2}$$

$$\Lambda' = \frac{\lambda}{2 \cdot n \cdot \cos(\psi' - \alpha')}$$

Beim Auslesen des Hologramms ("reconstruction") gilt wie analog oben dargestellt:

$$\vartheta'_0 = \theta_0 + \Omega$$
$$\sin(\vartheta'_0) = n \cdot \sin(\vartheta')$$

An der Bragg-Bedingung ist das "Dephasing" DP = 0. Und es folgt entsprechend:

$$\alpha'_0 = \theta_0 + \Omega_{reconstruction}$$
$$\sin(\alpha'_0) = n \cdot \sin(\alpha')$$

Der noch unbekannt Winkel $\beta'$ kann aus dem Vergleich der Bragg-Bedingung des Interferenzfeldes beim Schreiben des Hologramms und der Bragg-Bedingung beim Auslesen des Hologramms ermittelt werden unter der Annahme, dass nur Dickenschwindung stattfindet. Dann folgt:

$$\sin(\beta') = \frac{1}{n} \cdot \left[\sin(\alpha_0) + \sin(\beta_0) - \sin(\theta_0 + \Omega_{reconstruction})\right]$$

$\nu$ ist die Gitterstärke, $\xi$ ist der Detuning Parameter und $\psi'$ die Orientierung (Slant) des Brechungsindexgitters das geschrieben wurde. $\alpha'$ und $\beta'$ entsprechen den Winkeln $\alpha_0$ und $\beta_0$ des Interferenzfeldes beim Schreiben des Hologramms, aber im Medium gemessen und für das Gitter des Hologramms gültig (nach Dickenschwindung). n ist der mittlere Brechungsindex des Photopolymers und wurde zu 1.504 gesetzt. $\lambda$ ist die Wellenlänge des Laserlichts im Vakuum.

Die maximale Beugungseffizienz (DE = $\eta_{max}$) ergibt sich dann für $\xi$ = 0 zu:

$$DE = \tanh^2(\nu) = \tanh^2\left(\frac{\pi \cdot \Delta n \cdot d'}{\lambda \cdot \sqrt{\cos(\alpha') \cdot \cos(\alpha' - 2\psi)}}\right)$$

Die Messdaten der Beugungseffizienz, die theoretische Braggkurve und die transmittierte Intensität werden wie in Figur 4 gezeigt gegen den zentrierten Drehwinkel $\Delta\Omega \equiv \Omega_{reconstruction} - \Omega = \alpha'_0 - \vartheta'_0$, auch Winkeldetuning genannt, aufgetragen.

Da DE bekannt ist wird die Form der theoretischen Braggkurve nach Kogelnik nur noch durch die Dicke *d'* der Photopolymerschicht bestimmt. $\Delta$n wird über DE für gegebene Dicke *d'* so nachkorrigiert, dass Messung und Theorie von DE immer übereinstimmen. *d'* wird nun solange angepasst bis die Winkelpositionen der ersten Nebenminima der theoretischen Braggkurve mit den Winkelpositionen der ersten Nebenmaxima der transmittierten Intensität übereinstimmen und zudem die volle Breite bei halber Höhe (FWHM) für die theoretische Braggkurve und für die transmittierte Intensität übereinstimmen.

Da die Richtung in der ein Reflexionshologramm bei der Rekonstruktion mittels eines $\Omega$-Scans mitrotiert, der Detektor für das abgebeugte Licht aber nur einen endlichen Winkelbereich erfassen kann, wird die Braggkurve von breiten Holgrammen (kleines *d'*) bei einem $\Omega$-Scan nicht vollständig erfasst, sondern nur der zentrale Bereich, bei geeigneter Detektorpositionierung. Daher wird die zur Braggkurve komplementäre Form der transmittierten Intensität zur Anpassung der Schichtdicke *d'* zusätzlich herangezogen.

Figur 4 zeigt die Darstellung der Braggkurve $\eta$ nach der Coupled Wave Theorie (gestrichelte Linie), des gemessenen Beugungswirkungsgrades (ausgefüllte Kreise) und der transmittierten Leistung (schwarz durchgezogene Linie) gegen das Winkeldetuning $\Delta\Omega$.

Für eine Formulierung wurde diese Prozedur eventuell mehrfach für verschiedene Belichtungszeiten t an verschiedenen Medien wiederholt, um festzustellen bei welcher mittleren Energiedosis des einfallenden Laserstrahls beim Schreiben des Hologramms DE in den Sättigungswert übergeht. Die mittlere Energiedosis *E* ergibt sich wie folgt aus den Leistungen der zwei den Winkeln $\alpha_0$ und $\beta_0$ zugeordneten Teilstrahlen (Referenzstrahl mit $P_r$ = 0.50 mW und Signalstrahl mit $P_s$ = 0.63 mW), der Belichtungszeit t und dem Durchmesser der Irisblende (0.4 cm):

$$E\ (\mathrm{mJ/cm^2}) = \frac{2 \cdot [P_r + P_s] \cdot t\ (\mathrm{s})}{\pi \cdot 0.4^2\ \mathrm{cm}^2}$$

Die Leistungen der Teilstrahlen wurden so angepasst, dass in dem Medium bei den verwendeten Winkeln $\alpha_0$ und $\beta_0$, die gleiche Leistungsdichte erreicht wird.

In Beispielen wird jeweils der maximale Wert in $\Delta$n berichtet, die verwendeten Dosen liegen zwischen 4 und 64 mJ/$cm^2$ pro Arm.

## Verwendete Substanzen

CGI-909 (Tetrabutylammonium-tris(3-chlor-4-methylphenyl)(hexyl)borat, [1147315-11-4]) ist ein von der Fa. CIBA Inc., Basel, Schweiz, hergestelltes Versuchsprodukt.

Die eingesetzten (fluorierten) Alkohole und monofunktionellen Isocyanate wurden im Chemikalienhandel bezogen.

1,8-Diisocyanato-4-(isocyanatomethyl)octan (TIN) wurde wie in EP749958 beschrieben hergestellt.

2,4,4-Trimethylhexane-1,6-diisocyanat, Vestanat TMDI, ist ein Produkt der Evonik Degussa GmbH, Mar1, Deutschland.

### Herstellung der Polyol-Komponente:

In einem 1 L Kolben wurden 0.18 g Zinnoctoat, 374.8 g ε-Caprolacton und 374.8 g eines difunktionellen Polytetrahydrofuranpolyetherpolyols (Equivalentgewicht 500 g/Mol OH) vorgelegt und auf 120 °C aufgeheizt und so lange auf dieser Temperatur gehalten, bis der Festgehalt (Anteil der nicht-flüchtigen Bestandteile) bei 99.5 Gew.-% oder darüber lag. Anschließend wurde abgekühlt und das Produkt als wachsiger Feststoff erhalten.

### Herstellung des Urethanacrylats 1: Phosphorothioyltris(oxybenzol-4,1-diylcarbamoyl-oxyethan-2,1-diyl)trisacrylat

In einem 500 mL Rundkolben wurden 0.1 g 2,6-Di-tert.-butyl-4-methylphenol, 0.05 g Dibutylzinndilaurat (Desmorapid Z, Bayer MaterialScience AG, Leverkusen, Deutschland) sowie und 213.07 g einer 27 %-igen Lösung von Tris (p-isocyanatophenyl)thiophosphat in Ethylacetat (Desmodur® RFE, Produkt der Bayer MaterialScience AG, Leverkusen, Deutschland) vorgelegt und auf 60 °C erwärmt. Anschließend wurden 42.37 g 2-Hydroxyethylacrylat zugetropft und die Mischung weiter auf 60 °C gehalten, bis der Isocyanatgehalt unter 0.1 % gesunken war. Danach wurde abgekühlt und im Vakuum das Ethylacetat vollständig entfernt. Das Produkt wurde als teilkristalliner Feststoff erhalten.

### Herstellung des Urethanacrylats 2: 2-({[3-(Methylsulfanyl)phenyl]carbamoyl}oxy)-ethylprop-2-enoat):

In einem 100 mL Rundkolben wurden 0.02 g 2,6-Di-tert.-butyl-4-methylphenol, 0.01 g Desmorapid® Z, 11.7 g 3-(Methylthio)phenylisocyanat vorgelegt und auf 60 °C erwärmt. Anschließend wurden 8.2 g 2-Hydroxyethylacrylat zugetropft und die Mischung weiter auf 60 °C gehalten, bis der Isocyanatgehalt unter 0.1 % gesunken war. Danach wurde abgekühlt. Das Produkt wurde als hellgelbe Flüssigkeit erhalten

## Beispiel 1: Trifluorethyl-butylcarbamat

In einem 2 L Rundkolben wurden 0.50 g Desmorapid Z und n-Butylisocyanat 498 g vorgelegt und auf 60 °C erwärmt. Anschließend wurden 502 g Trifluorethanol zugetropft und die Mischung weiter auf 60 °C gehalten, bis der Isocyanatgehalt unter 0.1 % gesunken war. Danach wurde abgekühlt. Das Produkt wurde als farbloser Feststoff erhalten. Der nach Methode B bestimmte Brechungsindex $n_D^{20}$ beträgt 1.3900, der gemessene TGA95-Wert beträgt 63.7 ° C.

## Weitere Beispiele

Die Beispiele 2-25 wurden auf die für Beispiel 1 beschriebene Art und Weise in den in Tabelle 2 angegebenen Zusammensetzungen hergestellt Die zugehörigen $n_D^{20}$ und TGA95 Werte zu allen Beispielen wurden, wie in den obigen entsprechend benannten Abschnitten beschrieben, gemessen.

Tabelle 2: Herstellung und Charakterisierung der Beispiele 2-35

| Beispiel | Name | Isocyanat und Menge | Alkohol und Menge | Katalysator und Menge | Temp [°C] | Beschreibung |
|---|---|---|---|---|---|---|
| **2** | 2,2,2-Trifluorethyl-hexylcarbamat | n-Hexylisocyanat 55.9 g | Trifluorethanol 44.0 g | Desmorapid Z 0.05 g | 60 °C | farblose Flüssigkeit |
| **3** | 2,2,3,3-Tetrafluorpropyl-butylcarbamat | n-Butylisocyanat 10.7 g | 2,2,3,3-Tetrafluorpropan-1-ol 14.3 g | Desmorapid Z 0.01 g | 60 °C | farblose Flüssigkeit |
| **4** | Bis(2,2,2-trifluorethyl)-(2,2,4-trimethylhexan-1,6-diyl) biscarbamat | 2,4,4-Trimethylhexane-1,6-diisocyanat (TMDI) 496 g | Trifluorethanol 463 g | Desmorapid Z 0.48 G | 60 °C | farblose Flüssigkeit |
| **5** | Bis(2,2,2-trifluorethyl)-[4-({[(2,2,2-trifluorethoxy)-carbonyl]amino} methyl)octan-1,8-diyl]biscarbamat | 1,8-Diisocyanato-4-(isocyanatomethyl)octan (TIN) 228 g | Trifluorethanol 272 g | Desmorapid Z 0.48 g | 60 °C | farblose Flüssigkeit |
| **6** | 2,2,3,3,4,4,4-Heptafluorbutyl-butylcarbamat | n-Butylisocyanat 24.8 g | 2,2,3,3,4,4,4-Heptafluorbutanol 50.1 g | Desmorapid Z 0.04 g | 60 °C | farbloser Feststoff |
| **7** | 2,2,3,3,4,4,5,5,6,6,7,7,8,8,9,9-Hexadecafluornonyl-butylcarbamat | n-Butylisocyanat 186 g | 2,2,3,3,4,4,5,5,6,6,7,7,8, 8,9,9-Hexadecafluornonanol 813 g | Desmorapid Z 0.50 g | 60 °C | farblose Flüssigkeit |
| **8** | Bis(2,2,3,3,4,4,4-heptafluorbutyl)-(2,2,4-trimethylhexan-1,6-diyl) biscarbamat | 2,4,4-Trimethylhexane-1,6-diisocyanat (TMDI) 6.88 g | 2,2,3,3,4,4,4-Heptafluorbutanol 13.1 g | Desmorapid Z 0.01 g | 60 °C | farblose Flüssigkeit |
| **9** | Bis(2,2,3,3,4,4,4-heptafluorbutyl)-[4-({[(2,2,3,3,4,4,4-heptafluorbutoxy) carbonyl]amino}methyl)octan-1,8-diyl]biscarbamat | 1,8-Diisocyanato-4-(isocyanatomethyl)octan (TIN) 5.91 g | 2,2,3,3,4,4,4-Heptafluorbutanol 14.1 g | Desmorapid Z 0.01 g | 60 °C | farbloses Öl |
| **10** | 2,2,3,3,4,4,5,5,5-Nonafluorpentyl-butylcarbamat | n-Butylisocyanat 4.25 g | 2,2,3,3,4,4,5,5,5-Nonafluorpentan-1-ol 10.7 g | Desmorapid Z 0.02 g | 70 °C | farblose Flüssigkeit |

(fortgesetzt)

| Beispiel | Name | Isocyanat und Menge | Alkohol und Menge | Katalysator und Menge | Temp [°C] | Beschreibung |
|---|---|---|---|---|---|---|
| **11** | Bis(2,2,3,3,4,4,5,5,5-nonafluorpentyl)-(2,2,4-trimethylhexan-1, 6-diyl) biscarbamat | 2,4,4-Trimethylhexane-1,6-diisocyanat (TMDI) 4.43 g | 2,2,3,3,4,4,5,5,5-Nonafluorpentan-1-ol 10.6 g | Desmorapid Z 0.02 g | 70 °C | farblose Flüssigkeit |
| **12** | 2,2,3,3,4,4,5,5,5-Nonafluorpentyl-hexylcarbamat | n-Hexylisocyanat 5.05 g | 2,2,3,3,4,4,5,5,5-Nonafluorpentan-1-ol 9.94 g | Desmorapid Z 0.02 g | 70 °C | farblose Flüssigkeit |
| **13** | 2,2,3,3,4,4,5,5,5-Nonafluorpentyl-propan-2-ylcarbamat | i-Propylisocyanat 3.81 g | 2,2,3,3,4,4,5,5,5-Nonafluorpentan-1-ol 11.2 g | Desmorapid Z 0.02 g | 70 °C | farblose Flüssigkeit |
| **14** | 2,2,3,3,4,4,5,5,6,6,6-Undecafluorhexyl-butylcarbamat | n-Butylisocyanat 3.72 g | 2,2,3,3,4,4,5,5,6,6,6-Undecafluorhexan-1-ol 11.3 g | Desmorapid Z 0.02 g | 70 °C | farblose Flüssigkeit |
| **15** | Bis(2,2,3,3,4,4,5,5,6,6,6-undecafluorhexyl)-(2,2,4-trimethylhexan- 1,6-diyl) biscarbamat | 2,4,4-Trimethylhexane-1,6-diisocyanat (TMDI) 3.88 g | 2,2,3,3,4,4,5,5,6,6,6-Undecafluorhexan-1-ol 11.1 g | Desmorapid Z 0.02 g | 70 °C | farbloses Öl |
| **16** | 2,2,3,3,4,4,5,5,6,6,6-Undecafluorhexyl-hexylcarbamat | n-Hexylisocyanat 4.46 g | 2,2,3,3,4,4,5,5,6,6,6-Undecafluorhexan-1-ol 10.5 g | Desmorapid Z 0.02 g | 70 °C | farblose Flüssigkeit |
| **17** | Bis(2,2,3,3,4,4,5,5,6,6,7,7-dodecafluorheptyl)-(2,2,4-trimethylhexan-1, 6-diyl) biscarbamat | 2,4,4-Trimethylhexane-1,6-diisocyanat (TMDI) 3.60 g | 2,2,3,3,4,4,5,5,6,6,7,7-Dodecafluorheptan-1-ol 11.4 g | Desmorapid Z 0.02 g | 70 °C | farbloses Öl |
| **18** | 2,2,3,3,4,4,5,5,6,6,7,7-Dodecafluorheptyl-hexylcarbamat | n-Hexylisocyanat 4.15 g | 2,2,3,3,4,4,5,5,6,6,7,7-Dodecafluorheptan-1-ol 10.8 g | Desmorapid Z 0.02 g | 70 °C | farblose Flüssigkeit |
| **19** | 2,2,3,3,4,4,5,5,6,6,7,7-Dodecafluorheptyl-propan-2-ylcarbamat | i-Propylisocyanat 3.06 g | 2,2,3,3,4,4,5,5,6,6,7,7-Dodecafluorheptan-1-ol 11.93 g | Desmorapid Z 0.02 g | 70 °C | farblose Flüssigkeit |

(fortgesetzt)

| Beispiel | Name | Isocyanat und Menge | Alkohol und Menge | Katalysator und Menge | Temp [°C] | Beschreibung |
|---|---|---|---|---|---|---|
| **20** | 2,2,3,3,4,4,5,5,6,6,7,7-Dodecafluorheptyl-cyclohexylcarbamat | Cyclohexylisocyanat 4.10 g | 2,2,3,3,4,4,5,5,6,6,7,7-Dodecafluorheptan-1-ol 10.9 g | Desmorapid Z 0.02 g | 70 °C | farblose Flüssigkeit |
| **21** | 2,2,3,4,4,4-Hexafluorbutyl-butylcarbamat | n-Butylisocyanat 5.28 g | 2,2,3,4,4,4-Hexafluorobutan-1-ol 9.71 g | Desmorapid Z 0.02 g | 70 °C | farblose Flüssigkeit |
| **22** | 2,2,3,4,4,4-Hexafluorbutyl-hexylcarbamat | n-Hexylisocyanat 6.16 g | 2,2,3,4,4,4-Hexafluorobutan-1-ol 8.83 g | Desmorapid Z 0.02 g | 70 °C | farblose flüssigkeit |
| **23** | 2,2,3,4,4,4-Hexafluorbutyl-propan-2-ylcarbamat | i-Propylisocyanat 4.77 g | 2,2,3,4,4,4-Hexafluorobutan-1-ol 10.2 g | Desmorapid Z 0.02 g | 70 °C | farbloses Öl |
| **24** | 2,2,3,3,4,4,5,5-Octafluorpentyl-butylcarbamat | n-Butylisocyanat 4.48 g | 2,2,3,3,4,4,5,5-Octafluorpentan-1-ol 10.5 g | Desmorapid Z 0.02 g | 70 °C | farblose Flüssigkeit |
| **25** | Bis(2,2,3,3,4,4,5,5-octafluorpentyl)-{4-[({[(2,2,3,3,4,4,5,5-octafluorpentyl) oxy]carbonyl}amino)methyl]octan-1,8-diyl}biscarbamat | 1,8-Diisocyanato-4-(isocyanatomethyl)octan (TIN) 3.98 g | 2,2,3,3,4,4,5,5-Octafluorpentan-1-ol 11.0 g | Desmorapid Z 0.02 g | 70 °C | farbloses Öl |
| **26** | Bis(2,2,3,3,4,4,5,5-octafluorpentyl)-(2,2,4-trimethylhexan-1,6-diyl) biscarbamat | 2,4,4-Trimethylhexane-1,6-diisocyanat (TMDI) 4.67 g | 2,2,3,3,4,4,5,5-Octafluorpentan-1-ol 10.3 g | Desmorapid Z 0.02 g | 70 °C | farbloses Öl |
| **27** | 2,2,3,3,4,4,5,5-Octafluorpentyl-propan-2-ylcarbamat | i-Propylisocyanat 4.02 g | 2,2,3,3,4,4,5,5-Octafluorpentan-1-ol 10.9 g | Desmorapid Z 0.02 g | 70 °C | farblose Flüssigkeit |
| **28** | 2,2,3,3,4,4,5,5-Octafluorpentyl-cyclohexylcarbamat | Cyclohexylisocyanat 5.25 g | 2,2,3,3,4,4,5,5-Octafluorpentan-1-ol 9.73 g | Desmorapid Z 0.02 g | 70 °C | farblose Flüssigkeit |
| **29** | Bis(2,2,3,3-tetrafluorpropyl)-[4-({[(2,2,3,3-tetrafluorpropoxy) carbonyl]amino}methyl)octan-1,8-diyl]biscarbamat | 1,8-Diisocyanato-4-(isocyanatomethyl)octan (TIN) 5.83 g | 2,2,3,3-Tetrafluor-1-propanol 9.16 g | Desmorapid Z 0.02 g | 70 °C | farbloses Öl |

(fortgesetzt)

| Beispiel | Name | Isocyanat und Menge | Alkohol und Menge | Katalysator und Menge | Temp [°C] | Beschreibung |
|---|---|---|---|---|---|---|
| **30** | Bis(2,2,3,3-tetrafluorpropyl)-(2,2,4-trimethylhexan-1,6-diyl) biscarbamat | 2,4,4-Trimethylhexane-1,6-diisocyanat (TMDI) 6.64 g | 2,2,3,3-Tetrafluor-1-propanol 8.35 g | Desmorapid Z 0.02 g | 70 °C | farblose Flüssigkeit |
| **31** | 2,2,3,3-Tetrafluorpropyl-propan-2-ylcarbamat | i-Propylisocyanat 5.87 g | 2,2,3,3-Tetrafluorpropan-1-ol 9.11 g | Desmorapid Z 0.02 g | 70 °C | farblose Flüssigkeit |
| **32** | Ethyl-hexylcarbamat | n-Hexylisocyanat 36.7 g | Ethanol 13.3 g | Desmorapid Z 0.02 g | 60 °C | farblose Flüssigkeit |
| **33** | iso-Propyl-hexylcarbamat | n-Hexylisocyanat 34.0 g | iso-Propanol 16.0 g | Desmorapid Z 0.02 g | 60 °C | farblose Flüssigkeit |
| **34** | 3 Ethyl-butylcarbamat | n-Butylisocyanat 34.1 g | Ethanol 15.9 g | Desmorapid Z 0.02 g | 60 °C | farblose Flüssigkeit |
| **35** | iso-Propyl-butylcarbamat | n-Butylisocyanat 31.1 g | iso-Propanol 18.9 g | Desmorapid Z 0.02 g | 60 °C | farblose Flüssigkeit |

**Abschätzung des Brechungsindex ($n_D^{20}$) und der Flüchtigkeit (TGA95) von 2,2,2-Trifluorethyl-butylcarbamat - (Beispiel 1)**

Die dreidimensionale Struktur der vorgenannten Verbindung (Beispiel 1) wurde mit Hilfe der graphischen Benutzeroberfläche des Molecular Modeling Paketes *Materials Studio* der Firma Accelrys erzeugt und voroptimiert. Diese voroptimierte Struktur wurde dann als Input für die Konformerenanalyse verwendet, für welche ein Monte Carlo Algorithmus verwendet wurde, welcher, jeweils ausgehend vom grade zuvor erzeugten Konformer, alle Diederwinkel innerhalb des Moleküls nach dem Zufallsprinzip verändert. Auf diese Weise wurden 1000 Zufallskonformere erzeugt und direkt voroptimiert. Im Rahmen der Prozedur wurden alle Konfonnere direkt verworfen, welche eine relative Energie > 8.37 kJ/mol aufwiesen.

Im Anschluss an diese Prozedur wurde alle 90 erhaltenen Konformere unter Verwendung eines strengeren Konvergenzkriteriums nachoptimiert und dann einer Ähnlichkeitsanalyse unterzogen, wobei sehr ähnliche Konformere verworfen wurden. Die verbleibenden 34 Konformere wurden anschließend unter Verwendung des Quantenchemie-Pakets TURBOMOLE mit Hilfe der Dichtefunktionaltheorie geometrieoptimiert. Dabei wurden das B-P86 Dichtefunktional und der triple-ξ-Valenzbasissatz TZVP, welcher der TURBOMOLE Basissatzbibliothek entnommen wurde, verwendet und es wurde die COSMO Option, unter Verwendung der optimierten COSMO-Radien, eingeschaltet.

Im Folgenden wurden dann für die 22 Konformere deren relative DFT/COSMO Energy < 8 kJ/mol war entsprechend den Schritten e und f des erfindungsgemäßen Verfahrens die individuellen Deskriptioren $V_i$, $A_i$ und $M^2_i$ berechnet. Hierfür wurde das Programm COSMO*therm* der Firma COSMO*logic* verwendet. Die Ergebnisse sind in Tabelle 3 zusammengestellt.

Tabelle 3

| Konformer | Rel. Energie in kJ/mol | Boltzmann Gewicht in % | $V$(Å$^3$) | $A$(Å$^2$) | $M^2$ |
|---|---|---|---|---|---|
| 1 | 0.00 | 15.92 | 224.534 | 224.129 | 69.717 |
| 2 | 0.14 | 15.02 | 224.692 | 224.472 | 69.698 |
| 3 | 0.46 | 13.21 | 223.745 | 228.546 | 74.656 |
| 4 | 0.95 | 10.86 | 222.437 | 228.836 | 74.314 |
| 5 | 1.80 | 7.70 | 224.267 | 226.684 | 73.407 |
| 6 | 1.90 | 7.39 | 224.614 | 226.738 | 73.401 |
| 7 | 3.86 | 3.35 | 222.143 | 224.595 | 74.151 |
| 8 | 3.96 | 3.22 | 223.430 | 227.027 | 74.845 |
| 9 | 4.09 | 3.06 | 224.181 | 220.790 | 73.732 |
| 10 | 4.24 | 2.88 | 226.841 | 218.562 | 73.464 |
| 11 | 5.07 | 2.06 | 222.067 | 227.356 | 72.800 |
| 12 | 5.08 | 2.05 | 224.635 | 224.359 | 72.785 |
| 13 | 5.35 | 1.84 | 225.926 | 217.658 | 76.949 |
| 14 | 5.58 | 1.68 | 227.028 | 216.153 | 72.131 |
| 15 | 6.32 | 1.24 | 222.207 | 226.866 | 74.032 |
| 16 | 6.39 | 1.21 | 224.162 | 220.368 | 71.597 |
| 17 | 6.97 | 0.95 | 224.831 | 226.248 | 75.008 |
| 18 | 7.55 | 0.76 | 224.889 | 222.006 | 71.440 |
| 19 | 7.60 | 0.74 | 225.386 | 223.325 | 75.264 |
| 20 | 7.72 | 0.71 | 225.336 | 225.147 | 74.730 |
| 21 | 7.88 | 0.66 | 221.676 | 224.815 | 72.557 |
| 22 | 7.88 | 0.66 | 226.532 | 216.022 | 73.523 |

Als Boltzmann-gewichtete Mittelwerte ergeben sich daraus:
$A$ = 225.308 Å$^2$, $V$ = 224.137 Å$^3$, $M^2$ = 72.609

Damit ergeben sich für die Flüchtigkeit (TGA95) und die Dichte die Werte 85.0 °C und 1.197 g/mol. Die molare Polarisierbarkeit wurde mit Hilfe des 1986 von Crippen veröffentlichten QSPR-Ansatzes, welcher im *Materials Studio* QSAR Modul implementiert ist zu 40.310 m$^3$/mol abgeschätzt, woraus sich in Kombination mit der Dichte ein Brechungsindex von $n_D^{20} = 1.3999$ ergibt. Die Substanz ist damit mit einem TGA95 Wert von 85.0 °C und einem Brechungsindex von 1.3999 insgesamt im Sinne des erfindungsgemäßen Auswahlverfahrens nicht als Additiv für Photopolymer-Formulierungen geeignet, da ihre abgeschätzte Flüchtigkeit deutlich zu hoch ist. Wie der Vergleich mit den experimentellen Werten zeigt, ist diese Bewertung korrekt.

In analoger Weise wie fur Beispiel 1 wurden die Flüchtigkeiten und Brechungsindices der Beispiele 2-39 berechnet und die Ergebnisse in Tabelle 4 zusammengefasst. Darin enthalten sind 30 weitere fluorierte Urethane (Beispiele 2-31), 4 nicht-fluorierte Urethane (Beispiele 32-35), sowie vier kommerzielle Weichmacher (Beispiele 36-39)

Tabelle 4: Vergleich von Abgeschätzten und experimentellen Brechungsindices und TGA95-Werten.

| Beispiel | Name | V/Å$^3$ | A/Å$^2$ | M$^2$ | M/ (g/mol) | MP/ (m$^3$/mol) | ρ (QSPR) | $n_D^{20}$ (QSPR) | TGA95 (QSPR) | $n_D^{20}$ (Exp.) | TGA95 (Exp.) |
|---|---|---|---|---|---|---|---|---|---|---|---|
| 2 | 2,2,2-Trifluorethyl-hexylcarbamat | 266.609 | 268.162 | 76.273 | 227.23 | 49.51 | 1.146 | 1.4136 | 72.2 | 1.3984 | 72.5 |
| 3 | 2,2,3,3-Tetrafluorpropyl-butylcarbamat | 254.440 | 253.494 | 94.734 | 231.19 | 44.09 | 1.241 | 1.3893 | 86.5 | 1.3879 | 71.6 |
| 4 | Bis(2,2,2-trifluorethyl)-(2,2,4-trimethylhexan-1,6-diyl) biscarbamat | 449.384 | 412.813 | 142.624 | 410.35 | 83.03 | 1.285 | 1.4333 | 135.5 | 1.4202 | 167.1 |
| 5 | Bis(2,2,2-trifluorethyl)-[4-({[(2,2,2-trifluorethoxy)carbonyl]amino} methyl)-octan-1,8-diyl]biscarbamat | 571.236 | 540.825 | 214.491 | 551.40 | 103.39 | 1.384 | 1.4322 | 172.5 | 1.4213 | 174.9 |
| 6 | 2,2,3,3,4,4,4-Heptafluorbutylbutylcarbamat | 299.099 | 285.161 | 71.344 | 299.19 | 49.65 | 1.372 | 1.3727 | 75.5 | 1.3625 | 69.8 |
| 7 | 2,2,3,3,4,4,5,5,6,6,7,7,8,8,9,9-Hexadecafluornonyl-butylcarbamat | 476.888 | 415.268 | 92.904 | 531.23 | 72.10 | 1.569 | 1.3459 | 116.6 | 1.3555 | 111.8 |
| 8 | Bis(2,2,3,3,4,4,4-heptafluorbutyl)-(2,2,4-trimethylhexan-1,6-diyl) biscarbamat | 606.335 | 522.310 | 136.852 | 610.38 | 101.71 | 1.432 | 1.3930 | 151.5 | 1.3887 | 139.1 |
| 9 | Bis(2,2,3,3,4,4,4-heptafluorbutyl)-[4-({[(2,2,3,3,4,4,4-heptafluorbutoxy)-carbonyl]amino} methyl)octan-1,8-diyl]biscarbamat | 813.142 | 689.703 | 193.478 | 851.44 | 131.40 | 1.517 | 1.3846 | 191.3 | 1.3876 | 159.7 |
| 10 | 2,2,3,3,4,4,5,5,5-Nonafluorpentyl-butylcarbamat | 336.687 | 311.927 | 73.341 | 349.19 | 54.31 | 1.433 | 1.3640 | 83.5 | 1.3580 | 72.8 |

(fortgesetzt)

| Beispiel | Name | V/Å$^3$ | A/Å$^2$ | M$^2$ | M/ (g/mol) | MP/ (m$^3$/mol) | ρ (QSPR) | $n_D^{20}$ (QSPR) | TGA95 (QSPR) | $n_D^{20}$ (Exp.) | TGA95 (Exp.) |
|---|---|---|---|---|---|---|---|---|---|---|---|
| **11** | Bis(2,2,3,3,4,4,5,5,5-nonafluorpentyl)-(2,2,4-trimethylhexan-1,6-diyl) biscarbamat | 683.549 | 574.484 | 135.816 | 710.40 | 111.04 | 1.484 | 1.3807 | 160.5 | 1.3820 | 157 |
| **12** | 2,2,3,3,4,4,5,5,5-Nonafluorpentyl-hexylcarbamat | 379.492 | 352.267 | 73.741 | 377.25 | 63.52 | 1.369 | 1.3780 | 89.9 | 1.3690 | 83.5 |
| **13** | 2,2,3,3,4,4,5,5,5-Nonafluorpentyl-propan-2-ylcarbamat | 315.705 | 289.954 | 70.340 | 335.17 | 49.61 | 1.469 | 1.3541 | 79.0 | 1.3497 | 58.5 |
| **14** | 2,2,3,3,4,4,5,5,6,6,6-Undeca fluorhexyl-butylcarbamat | 374.581 | 335.864 | 68.869 | 399.20 | 58.98 | 1.479 | 1.3560 | 87.7 | 1.3538 | 82.6 |
| **15** | Bis(2,2,3,3,4,4,5,5,6,6,6-undecafluorhexyl)-(2,2,4-trimethylhexan-1,6-diyl) biscarbamat | 757.048 | 613.121 | 134.098 | 810.41 | 120.38 | 1.536 | 1.3736 | 169.4 | 1.3750 | 152.7 |
| **16** | 2,2,3,3,4,4,5,5,6,6,6-Undecafluorhexylhexylcarbamat | 416.853 | 379.371 | 73.759 | 427.25 | 68.18 | 1.419 | 1.3704 | 96.4 | 1.3640 | 90 |
| **17** | Bis(2,2,3,3,4,4,5,5,6,6,7,7-dodecafluorheptyl)-(2,2,4-trimethylhexan-1,6-diyl) biscarbamat | 811.336 | 671.039 | 176.795 | 874.44 | 127.93 | 1.560 | 1.3739 | 187.5 | 1.3839 | 158.6 |
| **18** | 2,2,3,3,4,4,5,5,6,6,7,7-Dodecafluorheptyl-hexylcarbamat | 445.778 | 401.101 | 94.859 | 459.27 | 71.96 | 1.440 | 1.3690 | 112.1 | 1.3716 | 117.6 |
| **19** | 2,2,3,3,4,4,5,5,6,6,7,7-Dodeca fluorbeptyl-propan-2-ylcarbamat | 381.492 | 338.779 | 91.463 | 417.19 | 58.05 | 1.534 | 1.3470 | 103.0 | 1.3563 | 93.3 |
| **20** | 2,2,3,3,4,4,5,5,6,6,7,7-Dodecafluorheptyl-cyclohexylcarbamat | 430.049 | 369.790 | 83.268 | 457.25 | 69.90 | 1.491 | 1.3731 | 105.9 | 1.3830 | 123.7 |

(fortgesetzt)

| Beispiel | Name | V/Å$^3$ | A/Å$^2$ | M$^2$ | M/ (g/mol) | MP/ (m$^3$/mol) | ρ (QSPR) | $n_D^{20}$ (QSPR) | TGA95 (QSPR) | $n_D^{20}$ (Exp.) | TGA95 (Exp.) |
|---|---|---|---|---|---|---|---|---|---|---|---|
| **21** | 2,2,3,4,4,4-Hexafluorbutyl-butylcarbamat | 291.826 | 273.825 | 90.643 | 281.20 | 49.25 | 1.332 | 1.3829 | 90.0 | 1.3775 | 73.8 |
| **22** | 2,2,3,4,4,4-Hexafluorbutyl-hexylcarbamat | 335.639 | 313.390 | 92.270 | 309.25 | 5 8.45 | 1.271 | 1.3959 | 95.5 | 1.3876 | 89.5 |
| **23** | 2,2,3,4,4,4-Hexafluorbutyl- propan-2-ylcarbamat | 269.738 | 259.029 | 91.749 | 267.17 | 44.54 | 1.368 | 1.3733 | 87.7 | 1.3682 | 71.5 |
| **24** | 2,2,3,3,4,4,5,5-Octafluorpentyl-butylcarbamat | 330.134 | 303.188 | 88.913 | 331.20 | 53.42 | 1.393 | 1.3674 | 93.7 | 1.3731 | 80.5 |
| **25** | Bis(2,2,3,3,4,4,5,5-octafluorpentyl)-{4-[({[(2,2,3,3,4,4,5,5-octafluorpentyl)oxy]carbonyl} amino)methy 1]octan-1,8-diyl} biscarbamat | 895.878 | 763.826 | 254.799 | 947.50 | 142.73 | 1.552 | 1.3840 | 215.0 | 1.3955 | 172.5 |
| **26** | Bis(2,2,3,3,4,4,5,5-octafluorpentyl)-(2,2,4-trimethylhexan-1,6-diyl) biscarbamat. | 667.015 | 566.787 | 174.890 | 674.42 | 109.26 | 1.457 | 1.3880 | 172.4 | 1.3971 | 163.9 |
| **27** | 2,2,3,3,4,4,5,5-Octafluorpentyl-propan-2-ylcarbamat | 308.600 | 286.261 | 90.842 | 317.18 | 48.72 | 1.429 | 1.3577 | 92.3 | 1.3654 | 74.4 |
| **28** | 2,2,3,3,4,4,5,5-Octafluorpentyl-cyclohexylcarbamat | 355.202 | 319.977 | 87.653 | 357.24 | 60.56 | 1.400 | 1.3905 | 96.7 | 1.3977 | 110.5 |
| **29** | Bis(2,2,3,3-tetrafluorpropyl)-[4-({[(2,2,3,3-tetrafluorpropoxy)-carbonyl]amino) methyl)octan-1,8-diyl]biscarbamat | 668.003 | 606.741 | 275.686 | 647.45 | 114.73 | 1.417 | 1.4162 | 202.8 | 1.4240 | 197.9 |
| **30** | Bis(2,2,3,3-tetrafluorpropyl)-(2,2,4-trimethylhexan-1,6-diyl) biscarbamat | 517.127 | 461.959 | 184.103 | 474.39 | 90.59 | 1.313 | 1.4155 | 161.6 | 1.4229 | 185.3 |

(fortgesetzt)

| Beispiel | Name | V/Å$^3$ | A/Å$^2$ | M$^2$ | M/ (g/mol) | MP/ (m$^3$/mol) | ρ (QSPR) | $n_D^{20}$ (QSPR) | TGA95 (QSPR) | $n_D^{20}$ (Exp.) | TGA95 (Exp.) |
|---|---|---|---|---|---|---|---|---|---|---|---|
| **31** | 2,2,3,3-Tetrafluorpropyl-propan-2-ylcarbamat | 234.335 | 231.845 | 93.129 | 217.16 | 39.38 | 1.268 | 1.3770 | 85.2 | 1.3831 | 78.1 |
| **32** | Ethyl-hexylcarbamat | 241.340 | 247.593 | 72.851 | 173.26 | 48.81 | 0.941 | 1.4431 | 65.5 | 1.4376 | 83.9 |
| **33** | iso-Propyl-hexylcarbainat | 264.827 | 264.082 | 70.384 | 187.28 | 53.23 | 0.930 | 1.4413 | 67.9 | 1.4336 | 84.9 |
| **34** | 3 Ethyl-butylcarbamat | 197.717 | 207.505 | 71.325 | 145.20 | 39.61 | 0.960 | 1.4367 | 59.2 | 1.4313 | 66.1 |
| **35** | iso-Propyl-butylcarbamat | 220.538 | 224.943 | 68.619 | 159.23 | 44.03 | 0.946 | 1.4361 | 60.1 | 1.4284 | 63.9 |
| **36** | Propylencarbonat | 115.733 | 128.139 | 71.612 | 102.09 | 21.49 | 1.167 | 1.4059 | 64.3 | 1.4210 | 87 |
| **37** | Adipinsaeuredimethylester | 221.881 | 226.334 | 97.343 | 174.20 | 42.28 | 1.055 | 1.4256 | 86.5 | 1.4280 | 80.4 |
| **38** | Diethylenglycoldiacetat | 231.636 | 242.845 | 113.550 | 190.19 | 43.90 | 1.110 | 1.4261 | 97.9 | 1.4300 | 99.1 |
| **39** | Zitronensäuretriethylester | 328.385 | 305.298 | 134.859 | 276.28 | 64.18 | 1.174 | 1.4575 | 123.9 | 1.4420 | 131 |

**Vergleich zwischen experimentellen und abgeschätzten TGA95 und Brechungsindexwerten**

Der Vergleich zwischen den experimentellen und den nach dem erfindungsgemäßen Auswahlverfahren abgeschätzten Brechungsindices und TGA95 Werten der Beispiele 1-39 (Tabelle 4), welcher in den Korrelationsdiagrammen Figur 3 und Figur 4 veranschaulicht ist, zeigt deutlich die Eignung des Verfahrens. Die Brechungsindices konnten mit einer Standardabweichung von 0.0082 bestimmt werden, wobei der Maximalfehler über den gesamten Satz mit einem Absolutwert von 0.0155 für das Beispiel 39 ebenfalls sehr klein ist. Die Standardabweichung für die TGA95-Werte ergibt sich zu 15.8°C, bei einem absoluten Maximalfehler von 42.5 °C für das fluorierte Urethan Beispiel 25. Dieser vergleichsweise große Fehler ist jedoch nicht signifikant, da sowohl der experimentelle Wert von 172.5 °C als auch der abgeschätzte Wert von 215.0 °C deutlich oberhalb der Eignungsgrenze von 100 °C liegen. Die Verbindung wird also, trotz des Fehlers, vom erfindungsgemäßen Verfahren korrekterweise als geeignet als Additiv für Photopolymer-Formulierungen eingestuft. Allgemein kann festgestellt werden, dass sehr große Unterschiede zwischen vorhergesagtem und experimentellen TGA95 typischerweise bei vergleichsweise hohen Absolutwerten (typischerweise > 150 °C) auftreten, was auf eine beginnende Zersetzung der Substanzen zurückzuführen ist. Diese Tatsache bedeutet keine Einschränkung der Aussagekraft des erfindungsgemäßen Auswahlverfahrens, da der Schwellenwert für die Eignung einer Substanz mit 100°C typischerweise weit unterhalb der Zersetzungstemperatur üblicher Additivmoleküle ist.

**Selektion der Beispielmoleküle auf Basis des erfindungsgemäßen Verfahrens**

Die Brechungsindices der Beispiele 1-39 liegen alle unterhalb von 1.4600, In diesem Fall entscheiden also die nach dem erfindungsgemäßen Verfahren abgeschätzten TGA95 über die Eignung als Additive in Photopolymer-Formulierungen. In Tabelle 5 sind alle Beispiele zusammengefasst deren abgeschätzte Flüchtigkeit, gemäß TGA95, größer ist als 100°C.

Tabelle 5: Erfindungsgemäß selektierte Additive.

| Beispiel | $n_D^{20}$ (QSPR) | TGA95(QSPR) | $n_D^{20}$ (Exp.) | TGA95 (Exp.) |
|---|---|---|---|---|
| **4** | 1.4333 | 135.5 | 1.4202 | 167.1 |
| **5** | 1.4322 | 172.5 | 1.4213 | 174.9 |
| **7** | 1.3459 | 116.6 | 1.3555 | 111.8 |
| **8** | 1.3930 | 151.5 | 1.3887 | 139.1 |
| **9** | 1.3846 | 191.3 | 1.3876 | 159.7 |
| **11** | 1.3807 | 160.5 | 1.3820 | 157.0 |
| **15** | 1.3736 | 169.4 | 1.3750 | 152.7 |
| **17** | 1.3739 | 187.5 | 1.3839 | 158.6 |
| **18** | 1.3690 | 112.1 | 1.3716 | 117.6 |
| **19** | 1.3470 | 103.0 | 1.3563 | 93.3 |
| **20** | 1.3731 | 105.9 | 1.3830 | 123.7 |
| **25** | 1.3840 | 215.0 | 1.3955 | 172.5 |
| **26** | 1.3880 | 172.4 | 1.3971 | 163.9 |
| **29** | 1.4162 | 202.8 | 1.4240 | 197.9 |
| **30** | 1.4155 | 161.6 | 1.4229 | 185.3 |
| **39** | 1.4575 | 123.9 | 1.4420 | 131.0 |

Ausgewählt für eine experimentelle Untersuchung der holographischen Aktivität werden die Beispiele **7** und **17,** welche auf Grund ihres niedrigen Brechungsindexes und der ausreichend hohen Flüchtigkeit sehr leistungsfähig sein sollten. Als Gegenbeispiel wird die Verbindung **2** ausgewählt, welche mit einer abgeschätzten Flüchtigkeit von 72.2 °C deutlich außerhalb der Grenzen des erfindungsgemäßen Verfahrens liegt.

**Herstellung der Photopolymerformulierung für die Herstellung von holographischen Folien**

**Folienbeispiel 1:**

6.77 g der wie oben beschrieben hergestellten Polyol-Komponente wurden mit 4.00 g Phosphorothioyltris (oxybenzol-4,1-diylcarbamoyl-oxyethan-2,1-diyl)trisacrylat (Ur e-thanacrylat 1), 4.00 g 2-({[3-(Methylsulfanyl)phenyl] carbamoyl}oxy)propylprop-2-enoat (Urethanacrylat 2) 3.00 g 2,2,3,3,4,4,5,5,6,6,7,7,8,8,9,9-Hexadecafluornonyl-butylcarbamat (Beispiel 7), 0.02 g Fomrez UL 28 (Urethanisierungskatalysator, Handelsprodukt der Fa. Momentive Performance Chemicals, Wilton, CT, USA), 0.30 g CGI 909 (Versuchsprodukt der Fa. Ciba Inc, Basel, Schweiz), 0.03 g Neu Methylenblau, 0.06 g BYK 310 und 1.02 g N-Ethylpyrrolidon bei 60 °C gemischt, so dass eine klare Lösung erhalten wurde. Anschließend wurde auf 30 °C abgekühlt, 1.25 g Desmodur® N3900 (Handelsprodukt der Bayer MaterialScience AG, Leverkusen, DE, Hexandiisocyanat-basiertes Polyisocyanat, Anteil an Iminooxadiazindion mindestens 30 %, NCO-Gehalt: 23.5 %) zugegeben und erneut gemischt. Die erhaltene, flüssige Masse wird dann mittels Rakel auf eine 36 μm dicke Polyethylenterephthalatfolie appliziert, und für 4.5 Minuten bei 80 °C in einem Umlufttroclaier getrocknet. Anschließend wird die Photopolymerschicht mit einer 40 μm dicken Polyethylenfolie abgedeckt und aufgewickelt.

**Folienbeispiel 2:**

6.77 g der wie oben beschrieben hergestellten Polyol-Komponente wurden mit 4.00 g Phosphorothioyltris (oxybenzol-4,1-diylcarbamoyloxyethan-2,1-diyl)trisacrylat (U r ethan¬acrylat 1), 4.00 g 2-({[3-(Methylsulfanyl)phenyl) carbamoyl}oxy)propylprop-2-enoat (Urethanacrylat 2) 3.00 g Bis(2,2,3,3,4,4,5,5,6,6,7,7-dodecafluorheptyl)-(2,2,4-trimethylhexan-1,6-diyl)biscarbamat (Beispiel 17), 0.02 g Fomrez UL 28 (Urethanisierungskatalysator, Handelsprodukt der Fa. Momentive Perfonnance Chemicals, Wilton, CT, USA), 0.30 g CGI 909 (Versuchsprodukt der Fa. Ciba Inc, Basel, Schweiz), 0.03 g Neu Methylenblau, 0.06 g BYK 310 und 1.02 g N-Ethylpyrrolidon bei 60 °C gemischt, so dass eine klare Lösung erhalten wurde. Anschließend wurde auf 30 °C abgekühlt, 1.25 g Desmodur® N3900 (Handelsprodukt der Bayer MaterialScience AG, Leverkusen, DE, Hexandiisocyanat-basiertes Polyisocyanat, Anteil an Iminooxadiazindion mindestens 30 %, NCO-Gehalt: 23,5 %) zugegeben und erneut gemischt. Die erhaltene, flüssige Masse wird dann mittels Rakel auf eine 36 μm dicke Polyethylenterephthalatfolie appliziert, und für 4.5 Minuten bei 80 °C in einem Umlufttrockner getrocknet. Anschließend wird die Photopolymersc.hicht mit einer 40 μm dicken Polyethylenfolie abgedeckt und aufgewickelt.

**Vereleichs-Folienbeispiel I:**

6.77 g der wie oben beschrieben hergestellten Polyol-Komponente wurden mit 4.00 g Phosphorothioyltris (oxybenzol-4,1-diylcarbamoyl¬oxyethan-2,1-diyl)trisacrylat (U r e-thanacrylat 1), 4.00 g 2-({[3-(Methylsulfanyl)phenyl] carbamoyl}oxy)propylprop-2-enoat (Urethanacrylat 2) 3.00 g 2,2,2-Trifluorethyl-butylcarbamat (Beispiel 1), 0.02 g Fomrez UL 28 (Urethanisierungskatalysator, (Handelsprodukt der Fa. Momentive Performance Chemicals, Wilton, CT, USA), 0.30 g CGI 909 (Versuchsprodukt der Fa. Ciba Inc, Basel, Schweiz), 0.03 g Neu Methylenblau, 0.06 g BYK 310 und 1.02 g N-Ethylpyrrolidon bei 60 °C gemischt, so dass eine klare Lösung erhalten wurde. Anschließend wurde auf 30 °C abgekühlt, 1.25 g Desmodur® N3900 (Handelsprodukt der Bayer MaterialScience AG, Leverkusen, DE, Hexandiisocyanat-basiertes Polyisocyanat, Anteil an Iminooxadiazindion mindestens 30 %, NCO-Gehalt: 23,5 %) zugegeben und erneut gemischt. Die erhaltene, flüssige Masse wird dann mittels Rakel auf eine 36 μm dicke Polyethylenterephthalatfolie appliziert, und für 4.5 Minuten bei 80 °C in einem Umlufttrockner getrocknet. Anschließend wird die Photopolymerschicht mit einer 40 μm dicken Polyethylenfolie abgedeckt und aufgewickelt.

Tabelle 6: Holographische Bewertung Folienbeispiele

| Folienbeispiel | Beispiel, [Gew.-%] | Δn |
|---|---|---|
| 1 | 7, 15 | 0.038 |
| 2 | 17, 15 | 0.032 |
| Vergleichs-Folienbeispiel | Beispiel, [Gew.-%] | Δn |
| 1 | 2, 15 | 0.012 |

Die beschriebenen Werte für Δn wurden bei Dosen von 4-32 mJ/cm$^2$ erzielt.

Die gefundenen Werte für die holographischen Eigenschaft Δn der holographischen Medien zeigen, dass die nach dem erfindungsgemäßen Verfahren ausgewählten Additive besser für die Verwendung in holographischen Photopolymer-Filmen geeignet sind.

**Patentansprüche**

1. Verfahren zur Auswahl von Verbindungen, die als Weichmachern in Photopolymer-Formulierungen zur Herstellung von hellen holographischen Medien verwendet werden können, bei dem

a) eine zu prüfende Verbindung ausgewählt wird,
b) unter Verwendung eines geeigneten Computerprogramms, wie zum Beispiel dem *conformers* Modul des Programmpaketes *Materials Studio* der Firma Accelrys, eine Konformerenanalyse der zu prüfenden Verbindung durchgeführt wird,
c) eine Geometrieoptimierung aller generierten Konformere mit Hilfe eines Kraftfeldverfahrens durchgeführt wird und der Konformerenraum anschließend mit Hilfe einer Ähnlichkeitsanalyse weiter reduziert wird,
d) eine quantenchemische Geometrieoptimierung der nach der Kraftfeldoptimierung energetisch günstigsten Konformere, unter Verwendung des B-P86 Dichtefunktionals und eines Triple-ζ-Valenzbasissatzess, sowie des *Conductor Like Screening Model* (COSMO) in Verbindung mit den optimierten COSMO-Radien oder, wenn diese für ein gegebenes Element nicht existieren, des 1.17-fachen des Bondi-Valenzradius, erfolgt,
e) die Fläche (A) in Å$^2$ und das eingeschlossene Volumen (V) in Å$^3$ der als Ergebnis der quantenchemischen Geometrieoptimierung erhaltenen dreidimensionalen COSMO-Abschirmladungsdichtenoberflächen der energetisch niedrigsten Konformere berechnet werden,
f) die COSMO-Abschirmladungsdichteoberflächen der energetisch niedrigsten Konformere mit Hilfe eines geeigneten Software-Paketes, wie z.B. dem Programm COSMO*therm* der Firma COSMO*logic,* in Segmente aufgeteilt werden, die mittleren Oberflächenabschirmladungsdichten (σ) dieser Segmente in Form einer Häufigkeitsverteilung P(σ) aufgetragen werden und die zweiten Momente ($M^2$) dieser Verteilung, definiert gemäß der Gleichung:

$$M^2 = 10 \cdot \sum_i P(\sigma_i) \cdot \sigma_i^2 \cdot \Delta\sigma$$

ermittelt werden, wobei Δσ die Intervallbreite der diskreten Häufigkeitsverteilung ist und die Ladungsdichten σ in der Einheit e/nm$^2$ angegeben werden,
g) die Volumina, Flächen und zweiten Momente aller betrachteten Konformere gemäß ihres Gewichtes in der Boltzmann Verteilung auf Basis der aus den quantenchemischen Geometrieoptimierungen gewonnenen Energien gemittelt werden,
h) die Flüchtigkeit der Substanz nach der Rechenvorschrift:

$$TGA95 \approx 207.015 \cdot \frac{M^2}{A} + 41.405 \cdot \sqrt[3]{V} - 253.2$$

abgeschätzt wird,
i) die Dichte der Verbindung bei Raumtemperatur mit Hilfe der Gleichung:

$$\rho = 0.89 \cdot \frac{M}{V \cdot N_A} - 0.2 \cdot \frac{A}{V} + 0.01 \cdot \sqrt{M^2}$$

abgeschätzt wird, wobei ρ die Dichte des Reinstoffes in g/cm$^3$, *M* die molare Masse in g/mol, $N_A$ die Avogadro-Zahl, *A* die COSMO-Abschirmladungsdichtenoberfläche in Å$^2$, *V* das von dieser Oberfläche umschlossene Volumen in Å$^3$ und $M^2$ das zweite Moment der Oberflächenabschirmladungsdichten-Häufigkeitsverteilung ist,
j) die abgeschätzte Dichte genutzt wird, um mit Hilfe der Lorentz-Lorenz Gleichung:

$$n_D = \sqrt{\frac{2 \cdot \frac{\rho \cdot MP}{M} + 1}{1 - \frac{\rho \cdot MP}{M}}}$$

die, nach einem möglichst genauen QSPR Ansatz abgeschätzte, molare Polarisierbarkeit (*MP*) in einen Brechungsindex bei 589 nm $(n_D^{20})$ umzurechnen,

k) ermittelt wird, ob die Flüchtigkeit der zur prüfenden Verbindung > 100°C und deren Brechungsindex ≤1.4600 ist, wobei die zu prüfende Verbindung als geeignet eingestuft wird, wenn beide Bedingungen erfüllt sind.

2. Verfahren nach Anspruch 1, **dadurch gekennzeichnet, dass** im Schritt d) eine quantenchemische Geometrieoptimierung des nach der Kraftfeldoptimierung energetisch niedrigsten Konformers, bevorzugt aller Konformere in einem Energiefenster von 0-4 kJ/mol und besonders bevorzugt aller Konformere in einem Energiefenster von 0-8 kJ/mol erfolgt.

3. Verfahren nach einem der Ansprüche 1 oder 2, **dadurch gekennzeichnet, dass** im Schritt k) geprüft wird, ob die Flüchtigkeit der zur prüfenden Verbindung > 120 °C und deren Brechungsindex nn ≤ 1.4500, bevorzugt ≤ 1.4400 besonders bevorzugt ≤1.4300 ist.

4. Photopolymer-Formulierung umfassend Matrixpolymere, Schreibmomomere und Photoinitiatoren, **dadurch gekennzeichnet, dass** sie wenigstens einen Weichmacher enthält, der nach einem Verfahren gemäß einem der Ansprüche 1 bis 3 ausgewählt ist , die Matrixpolymere Polyurethane sind und die Photopolymer-Formulierung zusätzlich Urethane als Weichmacher enthält.

5. Photopolymer-Formulierung nach Anspruch 4, **dadurch gekennzeichnet, dass** die Photoinitiatoren einen anionischen, kationischen oder neutralen Farbstoff und einen Coinitiator umfassen.

6. Photopolymer-Formulierung nach einem der Ansprüche 4 oder 5, **dadurch gekennzeichnet, dass** die Urethane mit wenigstens einem Fluoratom substituiert sind.

7. Photopolymer-Formulierung nach Anspruch 6, **dadurch gekennzeichnet, dass** die Urethane die allgemeine Formel (II)

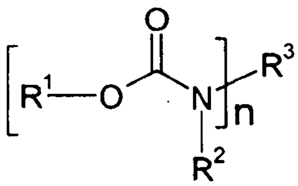

(II)

haben, in der n ≥1 und n ≤8 ist und $R^1$, $R^2$, $R^3$ Wasserstoff und / oder unabhängig voneinander lineare, verzweigte, cyclische oder heterocyclische unsubstituierte oder gegebenenfalls auch mit Heteroatomen substituierte organische Reste sind, wobei bevorzugt mindestens einer der Reste $R^1$, $R^2$, $R^3$ mit wenigstens einem Fluoratom substituiert ist und besonders bevorzugt $R^1$ ein organischer Rest mit mindestens einem Fluoratom ist.

8. Photopolymer-Formulierung nach einem der Ansprüche 4 bis 7, **dadurch gekennzeichnet, dass** die Schreibmonomere ein monofunktionelles Acrylat der allgemeinen Formel (IV)

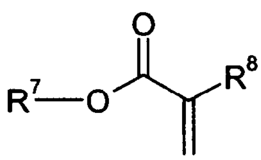

(IV)

umfassen, in der $R^7$, $R^8$ Wasserstoff und / oder unabhängig voneinander lineare, verzweigte, cyclische oder heterocyclische unsubstituierte oder gegebenenfalls auch mit Heteroatomen substituierte organische Reste sind.

**9.** Photopolymer-Formulierung nach einem der Ansprüche 4 bis 8, **dadurch gekennzeichnet, dass** die Schreibmonomere ein multifunktionales Schreibmonomer umfassen, wobei es sich insbesondere um ein multifunktionelles Acrylat, weiterhin bevorzugt ein multifunktionelles Urethanacrylat handeln kann.

**10.** Photopolymer-Formulierung nach Anspruch 9, **dadurch gekennzeichnet, dass** das multifunktionelle Acrylat die allgemeine Formel (V)

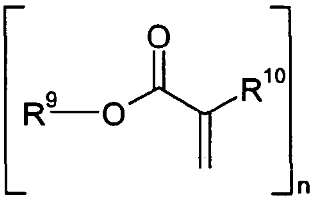

(V)

hat, in der n ≥2 und n ≤4 ist und $R^9$, $R^{10}$ Wasserstoff und / oder unabhängig voneinander lineare, verzweigte, cyclische oder heterocyclische unsubstituierte oder gegebenenfalls auch mit Heteroatomen substituierte organische Reste sind.

**11.** Verwendung einer Photopolymer-Formulierung nach einem der Ansprüche 4 bis 10 zur Herstellung holographischer Medien, insbesondere zur Herstellung von In-Line Hologrammen, Off-Axis Hologrammen, Full-Aperture Transfer Hologrammen, Weißlicht-Transmissionshologrammen, Denisyukhologrammen, Off-Axis Reflektionshologrammen, Edge-Lit Hologrammen sowie holographischen Stereogrammen.

## Claims

**1.** Method for selecting compounds which can be used as plasticizers in photopolymer formulations for the production of light holographic media, in which

a) a compound to be tested is selected,
b) a conformer analysis of the compound to be tested is carried out using a suitable computer programme, such as, for example, the *conformers* module of the *Materials Studio* programme package of Accelrys,
c) an optimization of the geometry of all conformers generated is carried out with the aid of a force field method and the conformer space is then further reduced with the aid of a similarity analysis,
d) a quantum chemical optimization of the geometry of the conformers which are energetically most favourable according to the force field optimization is effected with the use of the B-P86 density functional and a triple ζ valence basis set, and of the *Conductor Like Screening Model* (COSMO) in combination with the optimized COSMO radii or, if these do not exist for a given element, with 1.17 times the Bondi valence radius,
e) the area (A) in $Å^2$ and the enclosed volume (V) in $Å^3$ of the three-dimensional COSMO shielding charge density surfaces of the conformers having the lowest energy, which are obtained as result of the quantum chemical optimization of the geometry, are calculated,
f) the COSMO shielding charge density surfaces of the conformers having the lowest energy are divided into segments with the aid of a suitable software package, such, for example, the programme COSMO*therm* of COSMO*logic,* the mean surface shielding charge density (σ) of these segments are plotted in the form of a frequency distribution P(σ) and the second moments ($M^2$) of this distribution, defined according to the equation:

$$M^2 = 10 \cdot \sum_i P(\sigma_i) \cdot \sigma_i^2 \cdot \Delta\sigma$$

are determined, Δσ being the interval width of the discrete frequency distribution and the charge densities σ being stated in the unit $e/nm^2$,
g) the volumes, areas and second moments of all conformers considered are averaged according to their weight

in the Boltzmann distribution on the basis of the energies obtained from the quantum chemical optimizations of the geometries,
h) the volatility of the substance is estimated according to the computational rule:

$$TGA95 \approx 207.015 \cdot \frac{M^2}{A} + 41.405 \cdot \sqrt[3]{V} - 253.2$$,

i) the density of the compound at room temperature is estimated with the aid of the equation:

$$\rho = 0.89 \cdot \frac{M}{V \cdot N_A} - 0.2 \cdot \frac{A}{V} + 0.01 \cdot \sqrt{M^2}$$

ρ being the density of the pure substance in g/cm$^3$, *M* being the molar mass in g/mol, $N_A$ being the Avogadro number, *A* being the COSMO shielding charge density surface in Å$^2$, *V* being the volume in Å$^3$ enclosed by the surface and $M^2$ being the second moment of the surface shielding charge density frequency distribution,
j) the estimated density is used in order, with the aid of the Lorentz-Lorenz equation:

$$n_D = \sqrt{\frac{2 \cdot \frac{\rho \cdot MP}{M} + 1}{1 - \frac{\rho \cdot MP}{M}}}$$,

to convert the molar polarizability (MP) estimated according to a QSPR approach as accurately as possible into a refractive index at 589 nm $(n_D^{20})$
k) it is determined whether the volatility of the compound to be tested is > 100°C and the refractive index thereof is ≤ 1.4600, the compound to be tested being classed as being suitable if both conditions are fulfilled.

**2.** Method according to Claim 1, **characterized in that**, in step d), a quantum chemical optimization of the geometry of the conformer having the lowest energy according to the force field optimization, preferably of all conformers in an energy window of 0-4 kJ/mol and particularly preferably all conformers in an energy window of 0-8 kJ/mol, is effected.

**3.** Method according to either of Claims 1 and 2, **characterized in that**, in step k), a check is carried out to determine whether the volatility of the compound to be tested is > 120°C and the refractive index thereof $n_D$ is ≤ 1.4500, preferably ≤ 1.4400, particularly preferably ≤ 1.4300.

**4.** Photopolymer formulation comprising matrix polymers, writing monomers and photoinitiators, **characterized in that** it contains at least one plasticizer which is selected by a method according to any of Claims 1 to 3, the matrix polymers are polyurethanes, and the photopolymer formulation additionally contains urethanes as plasticizers.

**5.** Photopolymer formulation according to Claim 4, **characterized in that** the photoinitiators comprise an anionic, cationic or neutral dye and a coinitiator.

**6.** Photopolymer formulation according to either of Claims 4 and 5, **characterized in that** the urethanes are substituted by at least one fluorine atom.

**7.** Photopolymer formulation according to Claim 6, **characterized in that** the urethanes have the general formula (II)

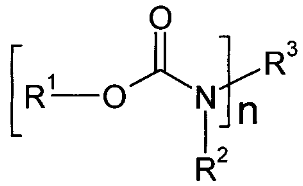

(II)

in which n is ≥ 1 and n is ≤ 8 and $R^1$, $R^2$, $R^3$ are hydrogen and/or, independently of one another, linear, branched, cyclic or heterocyclic organic radicals which are unsubstituted or optionally also substituted by heteroatoms, preferably at least one of the radicals $R^1$, $R^2$, $R^3$ being substituted by at least one fluorine atom and particularly preferably $R^1$ being an organic radical having at least one fluorine atom.

**8.** Photopolymer formulation according to any of Claims 4 to 7, **characterized in that** the writing monomers comprise a monofunctional acrylate of the general formula (IV)

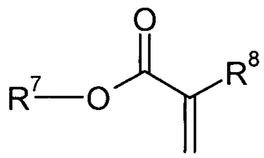

(IV)

in which $R^7$, $R^8$ are hydrogen and/or, independently of one another, linear, branched, cyclic or heterocyclic organic radicals which are unsubstituted or optionally also substituted by heteroatoms.

**9.** Photopolymer formulation according to any of Claims 4 to 8, **characterized in that** the writing monomers comprise a polyfunctional writing monomer, it being possible for this to be in particular a polyfunctional acrylate, more preferably a polyfunctional urethane acrylate.

**10.** Photopolymer formulation according to Claim 9, **characterized in that** the polyfunctional acrylate has the general formula (V)

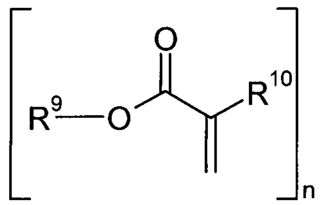

(V)

in which n is ≥ 2 and n is ≤ 4 and $R^9$, $R^{10}$ are hydrogen and/or, independently of one another, linear, branched, cyclic or heterocyclic organic radicals which are unsubstituted or optionally also substituted by heteroatoms.

**11.** Use of a photopolymer formulation according to any of Claims 4 to 10 for the production of holographic media, in particular for the production of in-line holograms, off-axis holograms, full-aperture transfer holograms, white light transmission holograms, Denisyuk holograms, off-axis reflection holograms, edge-lit holograms and holographic stereograms

**Revendications**

**1.** Procédé pour le choix de composés qui peuvent être utilisés comme plastifiants dans des formulations de photopolymère pour la préparation d'agents holographiques clairs, dans lequel

a) on choisit un composé à tester,
b) en utilisant un programme informatique approprié, tel que par exemple le module "*conformers*" du logiciel

*Materials Studio* de la société Accelrys, on réalise une analyse des conformères du composé à tester,
c) on réalise une optimisation de géométrie de tous les conformères générés à l'aide d'un procédé à champ de force et l'espace des conformères est ensuite réduit davantage à l'aide d'une analyse de similarité,
d) on effectue une optimisation de géométrie par chimie quantique des conformères énergétiquement les plus favorables d'après l'optimisation par champ de force, en utilisant la fonctionnelle de la densité B-P86 et un ensemble de bases triple-$\xi$ de valence, ainsi que le modèle COSMO (*Conductor Like Screening Model*) en association avec les rayons COSMO optimisés ou, lorsque ceux-ci n'existent pas pour un élément donné, avec le rayon de valence de Bondi augmenté d'un facteur 1,17,
e) on calcule la surface (A) en $Å^2$ et le volume enfermé (V) en $Å^3$ des surfaces de densité de charge de blindage COSMO tridimensionnelles obtenues comme résultat de l'optimisation de géométrie par chimie quantique des conformères énergétiquement les plus bas,
f) on répartit en segments les surfaces de densité de charge de blindage COSMO des conformères énergétiquement les plus bas à l'aide d'un logiciel approprié, comme par exemple le programme COSMO*therm* de la société COSMO*logic*, on reporte les densités de charge de blindage surfacique moyennes ($\sigma$) de ces segments sous forme d'une répartition de fréquences $P(\sigma)$ et on détermine les deuxièmes moments ($M^2$) de cette répartition, définis selon l'équation :

$$M^2 = 10 \cdot \sum_i P(\sigma_i) \cdot \sigma_i^2 \cdot \Delta\sigma$$

où $\Delta\sigma$ est la largeur de l'intervalle de la répartition des fréquences discrètes et les densités de charge $\sigma$ sont indiquées en unité e/nm$^2$,
g) on détermine les volumes, les surfaces et les deuxièmes moments de tous les conformères pris en considération en fonction de leur poids dans la répartition de Boltzmann sur base des énergies obtenues par les optimisations de géométrie par chimie quantique,
h) on estime la volatilité de la substance selon la règle de calcul :

$$TGA95 \approx 207.015 \cdot \frac{M^2}{A} + 41.405 \cdot \sqrt[3]{V} - 253.2$$

i) on estime la densité du composé à température ambiante à l'aide de l'équation :

$$\rho = 0.89 \cdot \frac{M}{V \cdot N_A} - 0.2 \cdot \frac{A}{V} + 0.01 \cdot \sqrt{M^2}$$

où p représente la densité de la substance pure en g/cm$^3$, M la masse molaire en g/mole, $N_A$ le nombre d'Avogadro, A la surface de densité de charge de blindage COSMO en $Å^2$, V le volume enfermé par cette surface en $Å^3$ et $M^2$ le deuxième moment de la répartition de fréquence des densités de charge de blindage surfacique,
j) on utilise la densité estimée pour convertir, à l'aide de l'équation de Lorentz-Lorenz :

$$n_D = \sqrt{\frac{2 \cdot \frac{\rho \cdot MP}{M} + 1}{1 - \frac{\rho \cdot MP}{M}}}$$

la polarisabilité molaire (PM) estimée selon une approche QSPR la plus précise possible en un indice de réfraction à 589 nm ($n_D^{20}$),
k) on détermine si la volatilité du composé à tester est > 100°C et son indice de réfraction est ≤ 1,4600, le composé à tester étant classé comme approprié lorsque les deux conditions sont remplies.

**2.** Procédé selon la revendication 1, **caractérisé en ce qu'**on réalise dans l'étape d) une optimisation de géométrie

par chimie quantique du conformère énergétiquement le plus bas selon l'optimisation par champ de force, de préférence de tous les conformères dans une fenêtre énergétique de 0-4 kJ/mole et de manière particulièrement préférée de tous les conformères dans une fenêtre énergétique de 0-8 kJ/mole.

**3.** Procédé selon l'une quelconque des revendications 1 ou 2, **caractérisé en ce qu'**on teste, dans l'étape k), si la volatilité du composé à tester est > 120°C et son indice de réfraction $n_D$ est $\leq$ 1,4500, de préférence $\leq$ 1,4400 de manière particulièrement préférée $\leq$ 1,4300.

**4.** Formulation de photopolymère comprenant des polymères de matrice, des monomères d'enregistrement et des photo-initiateurs, **caractérisée en ce qu'**elle contient au moins un plastifiant, qui est choisi selon un procédé selon l'une quelconque des revendications 1 à 3, les polymères de matrice sont des polyuréthanes et la formulation de photopolymère contient en outre des uréthanes comme plastifiants.

**5.** Formulation de photopolymère selon la revendication 4, **caractérisée en ce que** les photo-initiateurs comprennent un colorant anionique, cationique ou neutre et un co-initiateur.

**6.** Formulation de photopolymère selon l'une quelconque des revendications 4 ou 5, **caractérisée en ce que** les uréthanes sont substitués par au moins un atome de fluor.

**7.** Formulation de photopolymère selon la revendication 6, **caractérisée en ce que** les uréthanes présentent la formule générale (II)

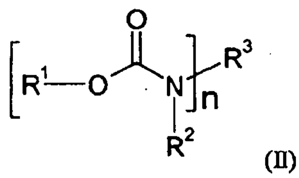

(II)

dans laquelle $n \geq 1$ et $n \leq 8$ et $R^1$, $R^2$, $R^3$ représentent hydrogène et/ou, indépendamment les uns des autres, des radicaux organiques linéaires, ramifiés, cycliques ou hétérocycliques, non substitués ou le cas échéant également substitués par des hétéroatomes, de préférence au moins un des radicaux $R^1$, $R^2$, $R^3$ étant substitué par au moins un atome de fluor et $R^1$ représentant de manière particulièrement préférée un radical organique comprenant au moins un atome de fluor.

**8.** Formulation de photopolymère selon l'une quelconque des revendications 4 à 7, **caractérisée en ce que** les monomères d'enregistrement comprennent un acrylate monofonctionnel de formule générale (IV)

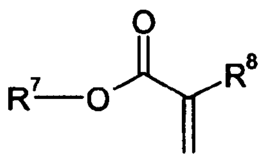

(IV)

dans laquelle $R^7$, $R^8$ représentent hydrogène et/ou, indépendamment l'un de l'autre, des radicaux organiques linéaires, ramifiés, cycliques ou hétérocycliques, non substitués ou le cas échéant également substitués par des hétéroatomes.

**9.** Formulation de photopolymère selon l'une quelconque des revendications 4 à 8, **caractérisée en ce que** les monomères d'enregistrement comprennent un monomère d'enregistrement multifonctionnel, où il peut en particulier s'agir d'un acrylate multifonctionnel, en outre de préférence d'un uréthane-acrylate multifonctionnel.

**10.** Formulation de photopolymère selon la revendication 9, **caractérisée en ce que** l'acrylate multifonctionnel présente la formule générale (V)

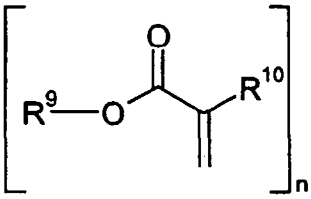

(V)

dans laquelle n ≥ 2 et n ≤ 4 et $R^9$, $R^{10}$ représentent hydrogène et/ou, indépendamment l'un de l'autre, des radicaux organiques linéaires, ramifiés, cycliques ou hétérocycliques, non substitués ou le cas échéant également substitués par des hétéroatomes.

**11.** Utilisation d'une formulation de photopolymère selon l'une quelconque des revendications 4 à 10 pour la production d'agents holographiques, en particulier pour la production d'hologrammes en ligne, d'hologrammes hors axe, d'hologrammes de transfert à pleine ouverture, d'hologrammes de transmission en lumière blanche, d'hologrammes de Denisyuk, d'hologrammes de réflexion hors axe, d'hologrammes à éclairage périphérique ainsi que de stéréogrammes holographiques.

Figur 1:

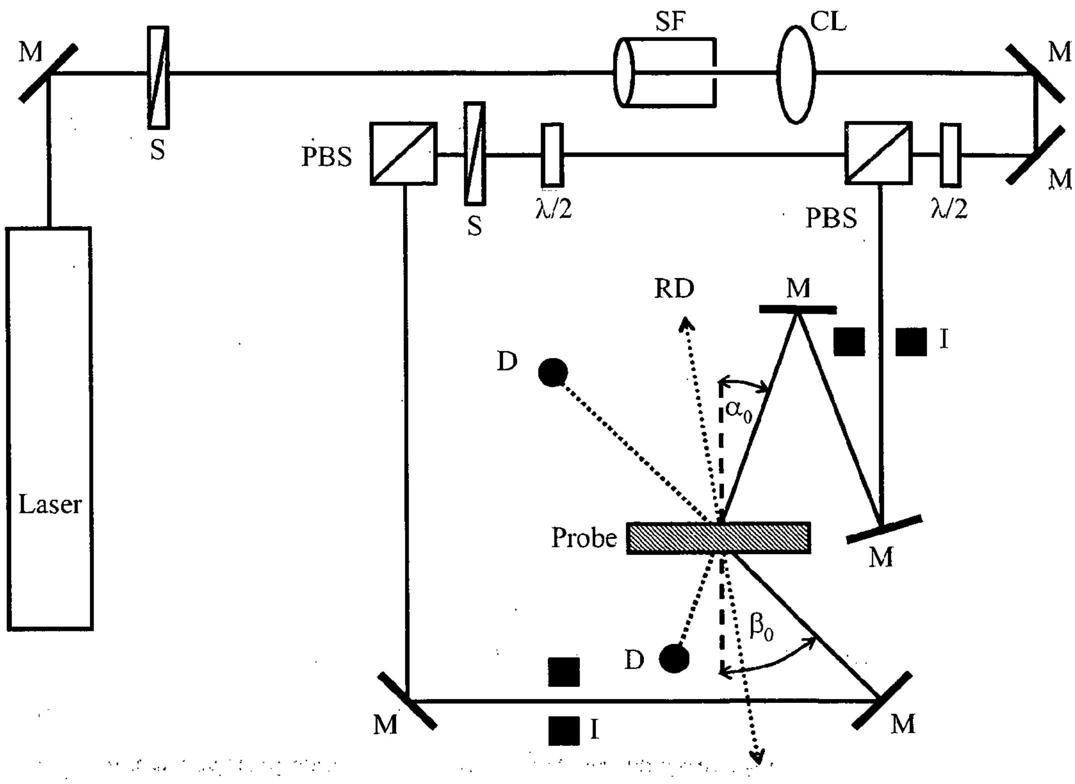

Figur 2:

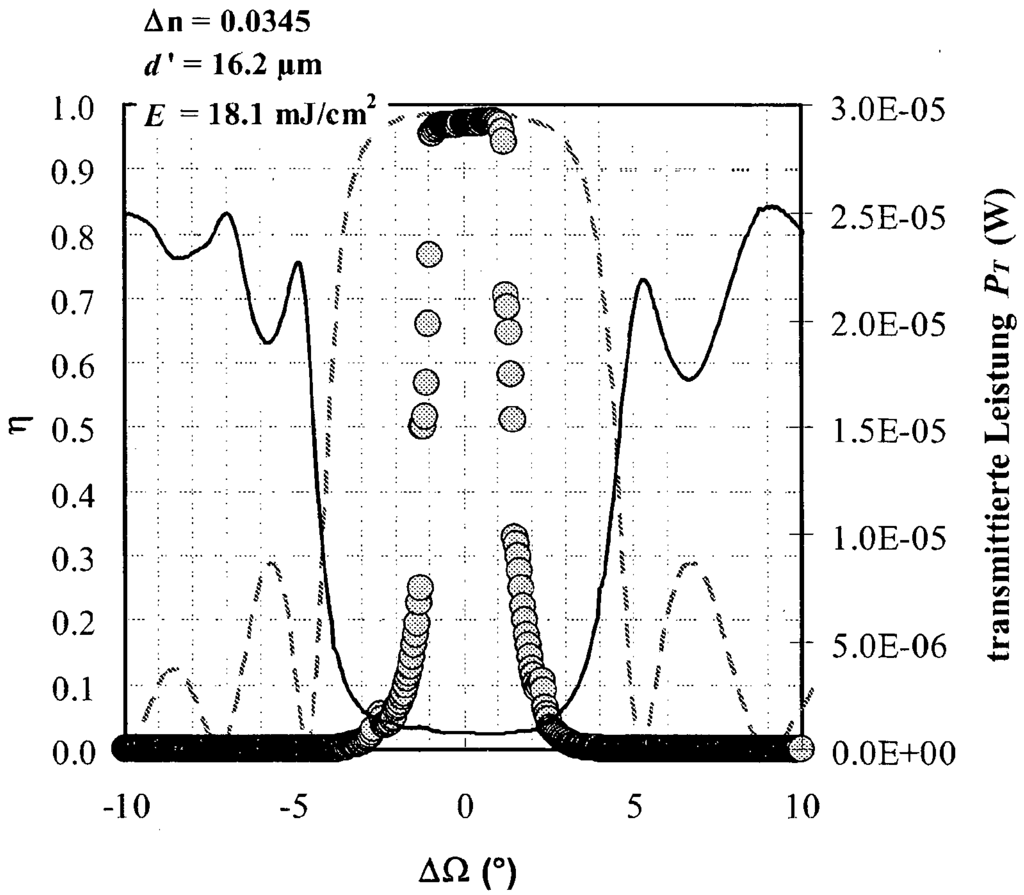
Δn = 0.0345
d' = 16.2 µm
E = 18.1 mJ/cm²
1.0
0.9
0.8
0.7
0.6
0.5
0.4
0.3
0.2
0.1
0.0
η
3.0E-05
2.5E-05
2.0E-05
1.5E-05
1.0E-05
5.0E-06
0.0E+00
transmittierte Leistung $P_T$ (W)
-10
-5
0
5
10
ΔΩ (°)

Figur 3

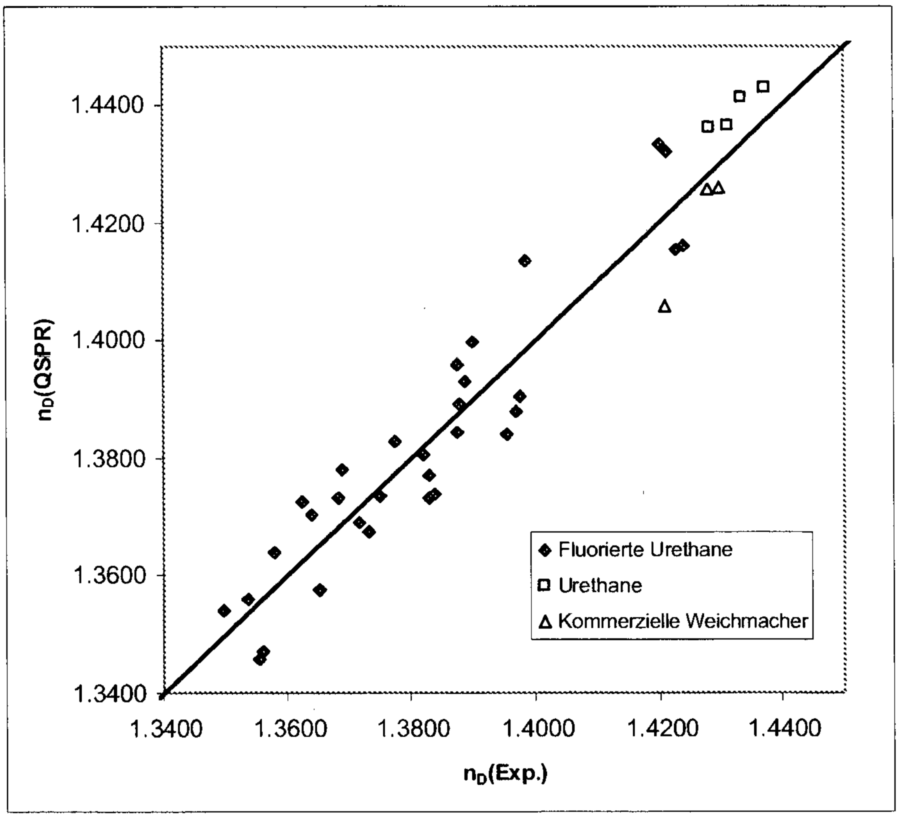
1.4400
1.4200
1.4000
1.3800
1.3600
1.3400
$n_D$(QSPR)
1.3400
1.3600
1.3800
1.4000
1.4200
1.4400
$n_D$(Exp.)
Fluorierte Urethane
Urethane
Kommerzielle Weichmacher

**Figur 4:**

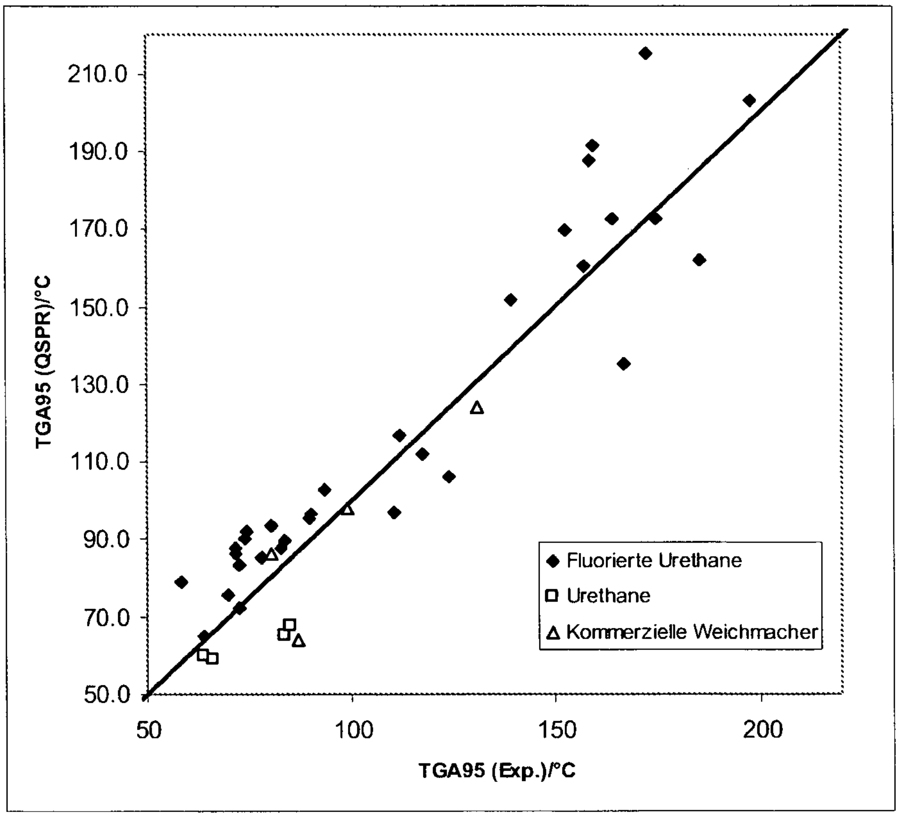
210.0
190.0
170.0
150.0
130.0
110.0
90.0
70.0
50.0
50
100
150
200
TGA95 (QSPR)/°C
TGA95 (Exp.)/°C
Fluorierte Urethane
Urethane
Kommerzielle Weichmacher

**Figur 5:**

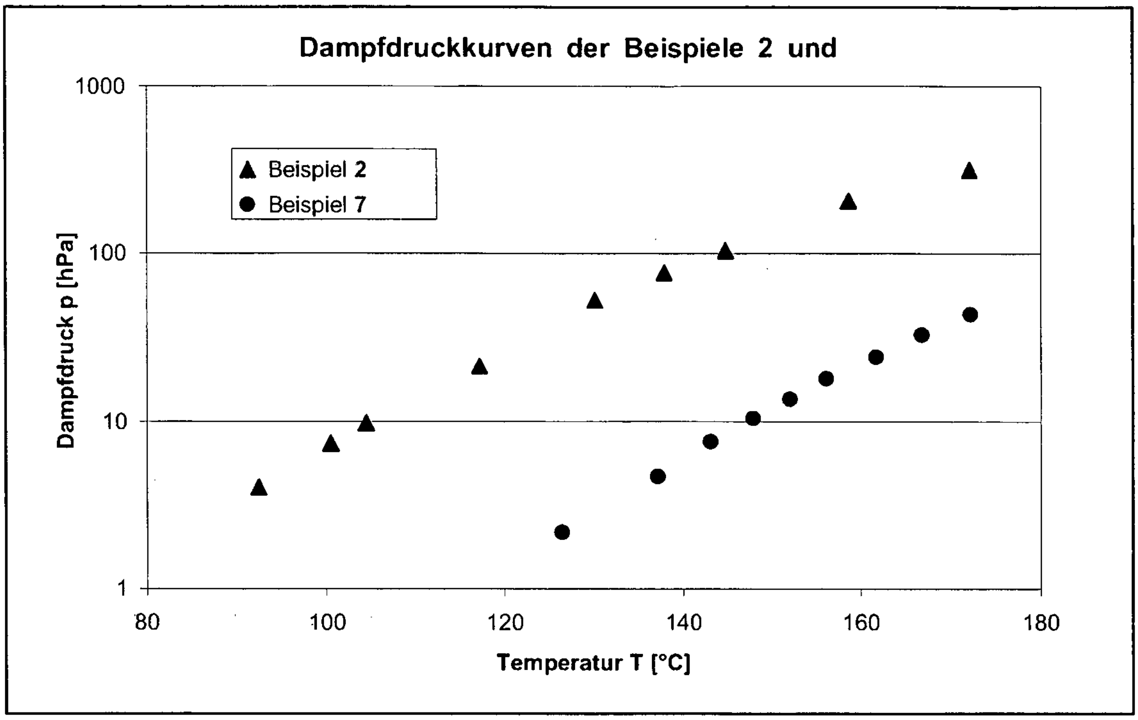
Dampfdruckkurven der Beispiele 2 und
Beispiel 2
Beispiel 7
Dampfdruck p [hPa]
1000
100
10
1
80
100
120
140
160
180
Temperatur T [°C]

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2008125229 A1
- WO 02051263 A
- DE 10065443
- EP 0223587 A
- EP 749958 A


### In der Beschreibung aufgeführte Nicht-Patentliteratur


- **VON HA et al.** *Energy & Fuels,* 2005, vol. 19, 152
- **J. MICRO ; NANOLITH.** *MEMS MOEMS,* 2008, vol. 7, 023001-1023001-11
- A QSPR for the plasticization efficiency of polyvinylchloride plasticizers. JOURNAL OF MOLECULAR GRAPHICS AND MODELLING. ELSEVIER SCIENCE, 06. Dezember 2007, vol. 26, 824-828
- **MARRERO ; GANI.** *Fluid Phase Equilib.,* 2001, vol. 183-184, 183-208
- *AIChE Journal,* 2002, vol. 48, 369
- *Fluid Phase Equilibria,* 2000, vol. 172, 43
- *J. Chem. Soc. Perkin Trans.,* 1993, vol. II, 799
- **CRIPPEN et al.** *J. Comput. Chem.,* 1986, vol. 7, 565
- *Chem. Inf. Comput. Sci.,* 1999, vol. 39, 868
- **CUNNINGHAM et al.** *RadTech'98 North America UV/EB Conference Proceedings, Chicago,* 19. April 1998
- **KUTAL et al.** *Macromolecules,* vol. 24, 6872
- **YAMAGUCHI et al.** *Macromolecules,* 2000, vol. 33, 1152
- **NECKERS et al.** *Macromolecules,* 2000, vol. 33, 7761
- **LI et al.** *Polymeric Materials Science and Engineering,* 2001, vol. 84, 139
- **DEKTAR et al.** *J. Org. Chem.,* 1990, vol. 55, 639
- *J. Org. Chem.,* 1991, vol. 56, 1838
- **GRIVELLO et al.** *Macromolecules,* 2000, vol. 33, 825
- **GU et al.** *Am. Chem. Soc. Polymer Preprints,* 2000, vol. 41 (2), 1266
- **HUA et al.** *Macromolecules,* 2001, vol. 34, 2488-2494
- Chemistry & Technology of UV & EB Formulations For Coatings, Inks & Paints. SITA Technology, 1991, vol. 3, 61-328
- **H. KOGELNIK.** *The Bell System Technical Journal,* November 1969, vol. 48 (9), 2909, 2947